(19) Europäisches Patentamt
European Patent Office
Office européen des brevets

(11) Publication number: **0 287 226**
**A1**

(12) # EUROPEAN PATENT APPLICATION

(21) Application number: 88302566.0

(22) Date of filing: 23.03.88

(51) Int. Cl.⁴: **A61K 39/21 , A61K 39/395 , A61K 39/42 , //G01N33/569**

The application is published incomplete as filed (Article 93 (2) EPC). The point in the description at which the omission obviously occurs has been left blank.

A request for addition of the omitted text has been filed pursuant to Rule 88 EPC. A decision on the request will be taken during the proceedings before the Examining Division (Guidelines for Examination in the EPO, A-V, 2.2).

(30) Priority: 23.03.87 GB 8706836
01.05.87 US 44716
01.06.87 GB 8712829
15.07.87 GB 8716632
30.10.87 GB 8725519

(43) Date of publication of application:
19.10.88 Bulletin 88/42

(84) Designated Contracting States:
AT BE CH DE ES FR GB GR IT LI LU NL SE

(71) Applicant: HIVER LIMITED
13 Southampton Place
London WC1A 2AJ(GB)

(72) Inventor: Dalgleish, Angus George
18 Lindfield Road
London W5 1QR(GB)
Inventor: Beverley, Peter Charles Leonard
111 Castlehaven Road
London NW1 8SJ(GB)
Inventor: Sattentau, Quentin James
5 Painsthorpe Road, Stoke Newington
London N16 0RB(GB)
Inventor: Kennedy, Ronald Curtis
2707 Indian Ridge Drive
San Antonio, Texas 78231(US)
Inventor: Chanh, Tran Cong
14814 Personality
San Antonio, Texas 78248(US)

(74) Representative: Ablewhite, Alan James et al
MARKS & CLERK 57/60 Lincoln's Inn Fields
London WC2A 3LS(GB)

(54) **Novel vaccines.**

(57) The present invention relates to vaccines against AIDS-like viruses comprising antibodies and their equivalents specific for such viruses or cellular receptors therefor, and to methods of treatment and prophylaxis using such vaccines.

EP 0 287 226 A1

## NOVEL VACCINES

The present invention relates to vaccines against Acquired Immune Deficiency Syndrome (AIDS) containing antibodies or their equivalent, and to the treatment and prophylaxis of AIDS using such vaccines.

AIDS was first recognised in the United States in the late 1970's when it was thought to be a rare phenomenon. However since then it has spread rapidly and has now achieved virtually pandemic proportions. The virus responsible principally infects cells of the immune system and particularly white blood cells carrying the CD4 or T4 receptor molecule, but it has recently been established that it can also infect cells of the nervous system and cells of the monocyte/macrophage lineage.

If AIDS develops after infection with the virus (human immunodeficiency virus (HIV)), the subject first becomes susceptible to opportunistic infections, due to the virus incapacitating the immune system through infection of the helper CD4 cells.

Various stages of infection have now been recognised. Progressive generalised lymphadenopathy (PGL) is characterised by general malaise and chronic cases of otherwise mild infections. AIDS related complex (ARC) is associated with general wasting and chronic illness while the condition known as AIDS is characterised by at least one bout of Pneumocystis carynii pneumonia and usually Kaposi's sarcoma.

While victims usually die of a severe form of an opportunistic infection(s), it is not impossible that the victim will die purely of HIV infection alone, as the virus has been proven capable of infecting the CNS, leading to death from neurological wasting.

HIV is now known as human immunodeficiency virus type 1 (HIV-1) (1,2) and is a retrovirus, related to the lentiviruses. The reason for the suffix of "1" is necessary because, since the original isolates were made, other related viruses have been discovered in West Africa. These new viruses were isolated from patients with an AIDS like disease (LAV-2) (3), or from healthy individuals whose sera cross-reacted with simian immunodeficiency virus (SIV) antigens (HTLV-IV) (4).

Both of these viruses have been shown to be closely related to SIV, and isolates have now been made from rhesus macaques (5), African green monkeys (6) and sooty mangabeys (7).

There is little cross-reactivity as measured by ELISA, Western blotting and immunoprecipitation assays between sera from patients with HIV-1 infection and HIV-2 or SIV antigens for the envelope glycoproteins, and similarly between sera from subjects with HIV-2, and HIV-1 envelope glycoprotein (3,4). Sera from HIV-2 infected subjects do show weak cross-neutralisation of HIV-1, which is concordant with the fact that there are relatively few conserved regions of sequence homology between the envelope regions of HIV-1 and HIV-2 (8). However, despite the general lack of cross-reactivity, HIV-1, HIV-2 and SIV are all tropic for the same families of lymphocytes.

Much effort has now been devoted to finding a cure, or a vaccine, for AIDS. Finding a cure has been hampered by the fact that HIV is a retrovirus and, once infection has occurred, it is impossible to totally eliminate the virus from the body. Thus, while efforts still continue in this direction, it is equally important that a vaccine be found to help prevent infection. Attempts to develop a vaccine have been hampered by the ability of the virus to alter the immunogenic determinants of its coat proteins.

A number of workers have recently focussed on the preparation of a subunit vaccine using the major envelope glycoprotein of HIV encoded by the env gene, either in a form purified from native virus, or as a recombinant protein from bacteria, vaccinia, SV40 viruses, or a mammalian cell line, or as synthetic peptides. This approach has yielded varied results. Immunisation of animals with some of these products elicited a specific antibody response which, in some cases, contained neutralising activity. However, the neutralising activity observed was relatively weak and appeared to be isolate-specific, a consequence of the aforementioned variation in the coat proteins between isolates.

The above, taken together with the fact that the neutralising antibody response in man is also weak, probably not protective and also, to some extent, isolate-specific, demonstrates an inability to produce a sufficiently protective immune response against the coat protein, presented either in its native form or as a recombinant or synthesised product.

The possibility of using anti-idiotype (anti-Id) antibodies, that is, antibodies having a binding site which recognises the binding site of the natural antibody, or idiotype (Id) raised against an antigen (Ag), has been suggested for a vaccine. This approach also suffers from the draw-back of variation in the env product as mentioned above. The advantage of using an anti-idiotype antibody in a vaccine is that it can mimic the receptor for the virus or the virus coat protein. However, the production of anti-idiotype antibodies (Ab-2), which exactly mirror the binding sites of conventional antibodies (Ab-1), is fraught with even more difficulties than the production of the Ab-1, as not only must the original antibody be produced which must in itself be useful (not so far possible), but the anti-idiotype must recognise only the binding site or paratope of the idiotype.

Attempts have been made to find an anti-idiotype antibody which recognises an HIV epitope, but such studies have failed (for example, McDougal, J.S., et al., The Journal of Immunology, (1986), 137, 2937-2944). Attempts to raise an Ab-1 recognising HIV, by using recombinant env have met with only limited success (Weiss, R.A., et al., Nature (1986), 324, 572-575) in that only certain strains of HIV are neutralised.

It has recently been found that HIV binds to any cell expressing the CD4 marker, and that antibodies against epitopes of this marker block the binding of the virus. Production of suitable antibodies in adequate quantities outside of the host is virtually impossible, and attempts to produce a vaccine stimulating the generation of such antibodies have not proven successful. The vaccines so far tried have comprised synthetic peptides mimicking the epitope of CD4 recognised by HIV. These probably fail because they do not adopt the correct configuration in situ.Recently, using CD4 monoclonal antibodies, we have mapped the epitopes on CD4 which are important in the binding of HIV-1 (12). The epitopes of CD4 involved in binding various highly divergent isolates of HIV-1 were shown to be very similar. We have now found that HIV-1, HIV-2 and certain isolates of SIV all use the CD4 antigen as the cellular receptor on human cells, that the epitopes in binding are very similar, and the expression of the receptor is downregulated following infection.

Immunoglobulin idiotypes (Id) have been studied extensively since they were first recognized on induced antibodies in humans and rabbits (Kunkel, H.G., et al. 1963. Science 140:1218; Oudin, J. et al. 1963. C.R. Seances Soc. Biol. 257:805). The network theory of immunoregulation encompassed the concepts regarding idiotypy and postulated that Id networks via Id-anti-Id reactions may be involved in regulating the immune system (Jerne. N.K. 1974. Ann. Immunol. 125C:373; Jerne, N.K. 1984. Imm. Rev. 79:5). By definition, an Id represents antigenic determinants associated with the variable (V) region of an antibody molecule. Thus, Id can be associated with sites involved in antigen binding (complementarity derived) and/or sites within the V region where antigen does not bind (framework). Id can be defined by serological counterparts referred to as anti-Id. Alternatively, Id and anti-Id have also been referred to as Ab-1 (first antibody) and Ab-2 (second antibody), respectively.

With retroviruses, as with other enveloped viruses, the antigenic determinants associated with the induction of neutralizing antibodies appear to reside with the surface envelope glycoproteins (Laskey, L.A., et al. 1986. Science 233:209; Robey, W.G., et al. 1986. Proc. Natl. Acad. Sci. USA 83:7023; Putney, S.D., et al. 1986. Science 234:1392; Ho, D.D., et al. 1987. J. Virol. 61:2024). The HIV envelope gene product is synthesized as a polyprotein precursor and is subsequently glycosylated within infected cells. This glycosylated polyprotein (molecular weight of 160kD [gp160]) is processed into an amino terminus subunit, gp120, and a carboxyl trans-membrane subunit gp41 (Muesing. M.A., et al. 1985. Nature 313:450; Robey, W.G., et al. 1985. Science 228:593).

Previous studies with anti-Id and hepatitis B virus have shown that internal image anti-Id (Ab-2$\beta$), which mimicked the original antigen, can induce an Id$^+$ anti-HBs$^+$ immune response which shares an Id with mouse anti-HBs produced by HBsAg (reviewed in Ellman, G.L. 1959. Arch. Biochem. Biophys. 82:70). In contrast, the anti-HBs induced by the Ab-2$\alpha$ expressed an Id that is not normally present during the immune response to HBsAg. Similar results have been reported in the TMV Id system, where an Ab-2$\alpha$ induced an anti-TMV response in BALB/c mice that shared an Id with the original rabbit anti-TMV used to generate the anti-Id (Francotta, M. et al. 1984. J. EXP. Med. 160:1485; Urbain, J., et al. 1984. Idiotype in Biology and Medicine, (G. Kohler, P.A. Cazenave and J. Urbain, eds.) Academic Press Inc., New York, p. 15; Francotta, M., et al. 1985. Proc. Natl. Acad. Sci. USA 82:8149). Thus, two different classes of anti-Id can induce anti-viral responses without exposure to or immunization with viral antigens.

Similar findings have been reported with Ab-2$\alpha$ and Ab-2$\beta$ inducing an anti-phosphorylcholine immune response in mice (Huang, J.-H., et al. 1986. J. Immunol. 137:770).

In a first aspect of the present invention there is provided a vaccine suitable for use in generating immunity against AIDS-type viruses, and comprising an antibody or antibodies, or idiotypic fragments thereof, or their equivalent, directed against either an AIDS-type virus or a cellular receptor therefor, and a non-toxic, pharmaceutically acceptable carrier.

In a further aspect of the present invention, there is provided a method for generating effective immunity against AIDS-type viruses in a human comprising administration of a vaccine as described above.

The vaccines according to the present invention are particularly suitable for the treatment and prophylaxis of AIDS-like diseases. This has not previously been possible for the reasons detailed above. Use of vaccines according to the present invention dispenses with the necessity for vaccination against each individual type of AIDS-related virus and provides universal protection against such viruses, overcoming the lack of immunogenicity in the native viral proteins.

In a yet further aspect of the present invention, there is provided a method of treatment of an HIV infection in a human comprising administration of an anti-AIDS effective amount of a vaccine as described herein.

The term "anti-AIDS", as used herein, means that amount of a substance, or combination of substances, suitable to substantially prevent or combat infection by HIV or other retrovirus causing at least one similar AIDS-like symptom in a human or other mammal. "Retroviral" pertains to those retroviruses responsible for immunodeficiency and especially those causing the manifestation of one or more symptoms normally associated with AIDS, in a human being or other mammal.

The 'AIDS-type' viruses are those viruses which rely upon recognition of the CD4 marker for infectivity. It will be appreciated that when the vaccines of the invention contain antibodies etc. which mimic or mirror the recognition epitope of CD4, then one vaccine is suitable to generate immunity to all the AIDS-type viruses The AIDS-type viruses targetted are preferably those infecting humans, particularly HIV-1, LAV-2 and HTLV-IV.

The term "mAb", as used herein, means an antibody, an idiotypic fragment thereof, or its equivalent, and also includes polyclonal antibodies, fragments and equivalents thereof, and, in any case, recognising an AIDS-type virus or a cellular receptor therefor. The term also includes those MAb's generated by CDR (Complementarity Dertermining Region) grafting,, such as would be obtained by grafting the CDRs from a mouse antibody to a human antibody. The method is described, for example, by Jones, P.T., et al in Nature, 321, 522-525 (1986). Fragments of mAb's are suitably the Fab and Fab'2 fragments, or cloned or synthetic sequences representing them.

Some epitopes of CD4 as recognised by CD4 mAbs are more important in binding and infection by AIDS-type viruses than others. This was established on the basis of the efficiency of blocking of syncytium formation by the mAbs, and their ability to cross-compete with one another. This pattern of inhibition is highly reproducible, for example with four divergent isolates of HIV-1.

In the accompanying Examples, CD4 mAbs fall into three clusters: (a) those which are efficient at blocking the HIV-CD4 interaction and do so irrespective of the virus isolate or type; (b) those 2 mAbs which consistently fail to block syncytium formation with any of the viruses or isolates; and (c) those 4 mAbs which are poor at blocking syncytia, and vary in their ability to block different isolates and types. These results demonstrate that very similar epitopes of CD4 are involved in binding env in all the viruses tested.

By implication, the binding site on the immunodeficiency viruses is also a highly conserved region. In general, sequence homology in the env gene of diverse isolates of HIV-1 varies by up to 30%. Greater diversity exists between HIV-1 and LAV-2, with only 41.7% of conserved sequence over the entire env gene (8). Data from studies in which a probe containing the env gene from LAV-1 was reacted with the genome of LAV-2 and STLV-III$_{mac}$ (SIC$_{MAC}$) revealed a lack of detectable hybridisation between these regions.

Similar results were obtained when the genome of STLV-III$_{agm}$ was probed with the env/LTR region of HIV-1 in which only a very weak reaction was seen. On the other hand, when a restriction map of HTLV-IV was compared with those of STLV-III$_{mac}$ and STLV-III$_{agm}$, very few differences were detected (15). These data are in accordance with the insignificant reactivity observed between the major envelope glycoprotein of HIV-1 and HIV-2 or SIV reactive antisera, or conversely between the env product of HIV-2 or SIV and HIV-1 antisera (3,4).

The implication of these findings is that there are no conserved regions of the env product to which substantial immune response is generated. Taken together with the finding in this study that the virus binding site for CD4 is highly conserved, it is clear that the binding site is not sufficiently immunogenic to provoke an antibody response.

This may be a result of a 'hidden' epitope which is only exposed at close proximity to CD4, masking of the epitope by glycosylation or salicylic acid, mimicking of self antigen, for example MHC-II, or a non-conformational epitope to which B-cells are unable to respond.

Thus, it is a particular advantage of the present invention that we provide Id's, anti-Id's and their equivalents which recognise a normally unavailable epitope on HIV, so providing protection.

A further advantage is the provision of the opposing Id's, anti-Id's and their equivalents which can induce those described above.

Modulation of CD4 antigen expression following expression of cells with HIV-1 has been described (9,10) and implicated in the pathogenesis of the virus in disease (16). It has also been shown that infection of CD4 bearing cells with a number of human or simian immunodeficiency viruses causes substantial downregulation of CD4.

All known AIDS-type viruses (with the possible exception of HTLV-IV) are known to cause an AIDS like syndrome in the natural hosts, it may be that the effect of drastically decreasing normal CD4 expression is significant in the pathogenesis of the virus infection.

In support of this supposition, complexing the gp120 of HIV-1 to CD4 has been shown to suppress T-cell activation in response to PHA in the target cells, implying that loss of functional epitopes or

4

internalisation of CD4 leads to the loss of certain immune functions.

Thus, it is preferable that the vaccines of the present invention are not targetted towards active binding of the CD4 marker.

It will be appreciated that mimotypes of the said antibodies can be used in accordance with the present invention. The term mimotype, as used herein, means a peptide, or peptide derivative, specifically synthesised to bind the paratope of a given antibody. Mimotypes may be prepared according to the methods of Geysen, H.M., et al. (PNAS, (1984), 81, 3998-4002 and PNAS (1985), 82, 178-182).

Rabbit antisera raised against a native antigenic determinant associated with gp41 neutralize HIV infectivity in vitro. The epitope has been identified by producing antisera in experimental animals against a synthetic peptide (mimotype) analogous to amino acid sequences 735 to 752 of the HTLV-IIIB isolate of HIV (Kennedy. R.C., et al. 1986. Science 231:1556.).

In a preferred embodiment, the present invention provides the generation of an anti-Id reagent recognizing an Id on antibodies raised against an HIV, especially a gp41, synthetic peptide.

Immunization with the anti-Id induces an anti-peptide response in BALB/c mice that binds a recombinant gp160 preparation. Serologically, the anti-Id induced mouse anti-HIV response, inhibits the Id-anti-Id reaction and expresses an Id different from mouse anti-HIV produced by immunization with the gp41 synthetic peptide. These data are indicative that Id networks are operational in modulating the immune response to HIV.

We demonstrate the production of anti-idiotypic antibodies (anti-Id)[3] to chimpanzee antibodies directed against a synthetic peptide corresponding to a native epitope associated with gp41 of human immunodeficiency virus (HIV) envelope glycoprotein in rabbits. The peptide was analogous to amino acid sequences 735 to 752 from the HTLV-IIIB isolate of HIV.

Immunization of BALB/c mice with the anti-Id induced an anti-peptide response which bound a recombinant gp160 preparation without subsequent peptide or gp 160 exposure.

Examples herein demonstrate that it is possible to induce a population of Id[+] antibodies with anti-HIV specificity by anti-Id immunization.

In addition, when the vaccines contain antibodies, or their equivalent, such as mimotypes, which mimic or mirror the CD4 recognition epitope, then boosting with recombinant env (for example from Genentech Inc., California, USA) in the form of a vaccine. Whilst priming with recombinant env will not work, once a response has been established, boosting will work.

The antibodies used may be either idiotypic or anti-idiotypic or antibodies generated therefrom directly or indirectly. Either type of antibody may be used in a vaccine to provide either passive or active immunity. Passive immunity is provided by the injection of a large enough dose of antibody to provide protection against AIDS in its own right. Active immunity can be obtained by vaccinating with an immunogenic amount of antibody to cause production of the anti-idiotype to the first antibody.

Several monoclonal antibodies (mAbs) have been raised against the CD4 marker, including OKT4a and Leu3a. These can be used in vaccines according to the invention. The anti-idiotype generated will thus bind to the HIV coat and particularly to the 120 kD env product (gp 120 or gp41). However, not all antiidiotypes raised against mAbs recognising the CD4 marker will necessarily bind to HIV, as HIV binds only one, or some, of the epitopes present on the CD4 molecule (Sattentau et al., Science, (1986), 234, 1120-1123).

Anti-idiotypes to such mAbs as Leu3a and OKT4a used as components of the above vaccines will generate anti-anti-idiotypes (Ab-3, also known as Ab-1'), recognising the CD4 and able to hinder HIV binding by blocking the receptor.

While any antibodies as described may be employed for use in accordance with the present invention, it is generally preferred that the immunity generated is directed against the virus rather than the cellular receptor. Thus, where anti-CD4 mAbs are used, it is preferred that the vaccine will be so prepared as to cause an antiidiotype response in the patient and, where an antiidiotype of an anti-CD4 mAb is administered, it is preferred that the vaccine will be so prepared as to confer passive immunity on the patient.

It will be appreciated that in one aspect, the present invention is particularly concerned with anti-idiotype antibodies. Where an anti-idiotype is used in a vaccine, this is with the intention of eliciting an anti-idiotype response. Where an anti-idiotype is used, this may be either to elicit an anti-anti-idiotype response, or as a passive vaccine.

In another aspect, the present invention is concerned with anti-idiotypes as above, but using engineered substances and antibodies capable of mimicking the effects described above.

In general, it is preferred that active immunity inducing vaccines contain an antibody component mirroring the CD4 marker, whilst passive vaccines contain antibody components that mimic the CD4 marker.

Administration of vaccines according to the present invention will vary according to the circumstances, taking into account such factors as age, weight and general condition of the patient.

The vaccine may be administered as one self-sufficient dose or as a series of doses over a period of time.

Repetition of dosing either to "boost" or maintain the immunity is also generally desirable at a later time, conveniently about 3 months later, but such booster dosing may be given earlier or at any time during the remainder of the life-time of the patient, and on as many occasions are necessary.

Pharmaceutical grade saline may be used as carrier for the antibody or antibodies, to provide a simple vaccine. However, as the anti-idiotype response to such a formulation is not usually very strong, it is preferred to use an adjuvant.

Particularly useful adjuvants and carrier proteins for use in accordance with the present invention are keyhole limpet haemocyanin (KLH) and alum preparations, examples of which are provided below. Use of these substances has been found to greatly enhance the Ab-2 response and so vaccines containing such substances form particularly preferred embodiments of the present invention. Antigens not bound to a carrier protein may be poorly immunogenic or even not work at all.

Where passive immunity is required, there is no requirement for an adjuvant. However, where an antiidiotype response is to be generated, an adjuvant, together with an immune-stimulating quantity of mAb, is particularly useful.

When it is desired to use the vaccines described herein prophylactically, then it is generally preferable to use those vaccines adapted to stimulate active immunity. Vaccines conferring passive immunity are still of prophylactic use, however, where there is an immediate risk of HIV-infection, or it is suspected that active immunity cannot be generated, or not generated sufficiently quickly.

Where the vaccines according to the present invention are for treatment of an HIV-infection, then it is generally preferable to employ those vaccines adapted to confer passive immunity, for an immediate anti-AIDS effect. Vaccines adapted to stimulate active immunity may still be employed, in these circumstances, for providing longer-term effective measures.

When an adjuvant is used, it may be administered together with mAb, in the same or different preparations or separately, at a time different from that of the administration of the vaccine.

Vaccines according to the present invention will usually be administered by a conventional route and may be administered, for example, by injection by the intraperitoneal, intramuscular or subcutaneous routes, via dermal abrasion or orally.

Such vaccines will normally comprise a pharmaceutically acceptable carrier and optionally an adjuvant, substances to render the vaccine isotonic with the body fluids and such flavouring, emulsifiers and other ingredients as may be required.

Vaccines conferring passive immunity will usually contain a large dose of mAb, suitable to confer passive immunity on the patient. Vaccines for generating an immune response will usually contain an immune-stimulating quantity of mAb, which is generally less that is required for passive immunity.

Such vaccines as described above may be sub-divided for separate administration, whether simultaneously or over a period of time, suitably weeks.

The following Examples serve to illustrate the invention only, and should not be construed as limiting it in any way.


EXAMPLE 1


Production and Screening of Anti-Idiotype Antisera in Mice.


A. Coupling of Monoclonal Antibody (MAb) to Keyhole Limpet Haemocyanin (KLH).

The MAb in the form of purified immunoglobulin (Ig) in a solution of phosphate buffered saline Dulbecco 'A' (PBS'A') was further diluted in PBS'A' to 1mg/ml. Keyhole limpet haemocyanin (KLH) was dissolved in PBS'A' at a concentration of 1mg per ml, and dialysed overnight against 100 volumes (vols) of PBS'A' at 4°C. One vol of the MAb solution was mixed with 1 vol of KLH solution, and the reagents coupled by the addition of 1% vol/vol of a solution of 28% glutaraldehyde. After 1hr at room temperature, the solution was dialysed against 100 vols of PBS'A' overnight at 4°C.

## B. Immunisation of Mice

### 2.1 Priming of Mice with MAb/KLH Complex.

The MAb/KLH complex at a concentration of 1mg per ml was emulsified in an equal volume of Freund's complete adjuvant (FCA) to yield a water in oil emulsion. Two hundred microlitres of the emulsion containing 100µg of the Mab/KLH complex was inoculated subcutaneously (SC) under the skin on the belly of each inbred Imperial Cancer Research Fund (ICRF) Balb/C mouse.

### 2.2 Boosting of Mice

Twentyeight days following the priming of the mice, 200µl of a water in oil emulsion of 100µg of MAb/KLH complex in an equal volume of Freunds incomplete adjuvant (FIA) was inoculated under the skin of the belly, and 200µl into the thigh muscle of the right hind leg.

After 10 days the mice were bled from the tail (yielding between 100 and 200µl of blood) and rested. Fourteen days after the first booster, each mouse was given a further booster of 200µl of solution of Mab/KLH, containing 200µg of the complex into the peritoneal cavity. Ten days later, the mice were tail bled as before, and given one further booster inoculation as described above. After a further 10 days, the mice were again tail bled, and rested.

## C. Testing of Sera for Anti-Idiotype Activity.

### (i) Plate Binding Radioimmunoassay

Monoclonal-Id-antibody at a concentration of 50µg per ml in PBS'A' was added to a 96-well Cooke plate at 50µl per well. After overnight incubation at 4°C, the plate was washed 3 times with PBS'A'/1% BSA, then incubated with a further 50µl per well of PBS'A'/1% BSA for 1 hour at 37°C. The PBS'A' was then replaced with the antisera from immune mice at dilutions of 1/10 or greater in PBS'A'/1% BSA. Negative controls were PBSA/BSA only, dilutions of pre-immune or normal mouse sera, and the positive control was polyvalent rabbit anti-mouse serum at 20µg per ml, or dilutions of this. The plate was incubated for 1 hr at 37°C, washed 3 times as before, and 2000 to 4000 specific counts per minute (CPM) of $^{125}$I-labelled Id was added in 50µl PBS'A'/BSA to each well. After a further 1 hr, the plate was again washed as above, the wells filled with wax, and the CPM determined for each well on a LKB ultrogamma.

The results were expressed as the percentage of bound $^{125}$I cross-linked by the test samples as compared to the maximum CPM for the positive control.

### (ii) Binding Inhibition Assay.

2000 to 5000 specific CPM of idiotype antibody labelled with $^{125}$I was mixed with antisera from immune mice at various dilutions, and incubated at 4°C for 1/2 hour in a 96-well Cooke plate. CD4 bearing CEM cells were then added at a concentration of $10^5$ cells per well, followed by a further incubation as above. The cells were washed 3 times in PBS'A'/1% BSA by centrifugation, the wells filled with wax, and CPM bound to the cells measured on an LKB ultrogamma.

## D. Screening Antisera for Virus-Specific Reactivity.

## Indirect Immunofluorescent Staining of Virus Infected Cells.

CEM cells uninfected or infected with HIV were incubated at a concentration of $10^5$ cells in 50μl of MEM with 2% FCS and 0.1% sodium azide per well of a 96-well microtitre plate for 1/2 hr at 4°C with 50μl of a 1/10 dilution of either immune antisera or normal mouse serum. The cells were washed 3 times by centrifugation through MEM/2% FCS/0.1% sodium azide, then resuspended in a 1/100 dilution of rabbit polyvalent anti-mouse Ig FITC conjugate (Sigma), and incubated as above. Finally, the cells were washed a further 3 times, resuspended in 200μl MEM/2% FCS/0.1% sodium azide at 4°C and the percentage fluorescence was determined by analysis on a Becton Dickinson FACS analyser.

## E. Testing Antisera for Virus Neutralising Activity.

### Inhibition of Virus Induced Syncytium Formation.

Virus specific syncytium formation is induced by the mixing of an HIV producing cell line (H9) with a CD4 bearing line (JM or C8166) in the ratio 1:5 in RPMI 1640 + 10% FCS, and incubating overnight at 37°C. Inhibition of syncytium formation, by blocking the CD4-HIV interaction either with CD4 MAbs, or antibodies directed against the envelope glycoprotein of the virus, can be measured. Specific inhibition of syncytium formation by a reagent reacting with the virus rather than the receptor can be demonstrated by a 2 part assay. The antisera were added at various dilutions to $10^5$ of the indicator cells in 50μl of MEM/FCS, and incubated for 1/2 hr at 4°C. The cells were washed 3 times and mixed with an HIV producing line at a ratio of 5:1. An equal number of CD4 bearing indicator cells were incubated with the antisera, and were mixed with the virus-producing cells without any washing. Blocking of syncytia in the second but not first assay indicates inhibition of the virus-receptor interaction at the virus, not the receptor end.

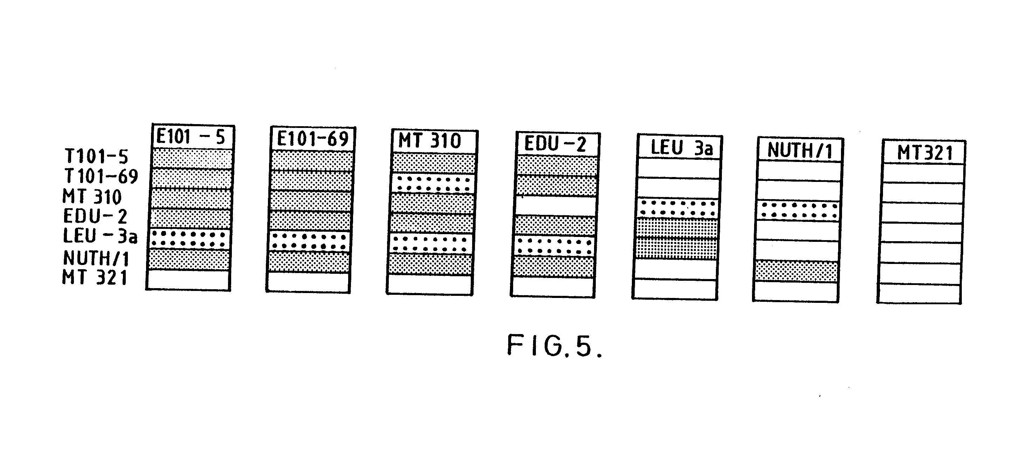

Antisera tested had anti-id activity detectable at dilutions of at least 1:250 or 1:200 respectively (Table 1). This activity was specific for the immunising Id MAb (the antisera did not bind to irrelevant antibodies, or to OKT4, a MAb recognising another CD4 epitope) and could be blocked by addition of the Id MAb to the antisera in the assay. Figure 5 shows cross-inhibition of binding of CD4 by anti-id sera. The left-hand column shows the anti-idiotype antisera. The boxes indicate the type of CD4 (idiotype) antibodies. Heavy shading denotes complete inhibition and part shading denotes partial inhibtion..

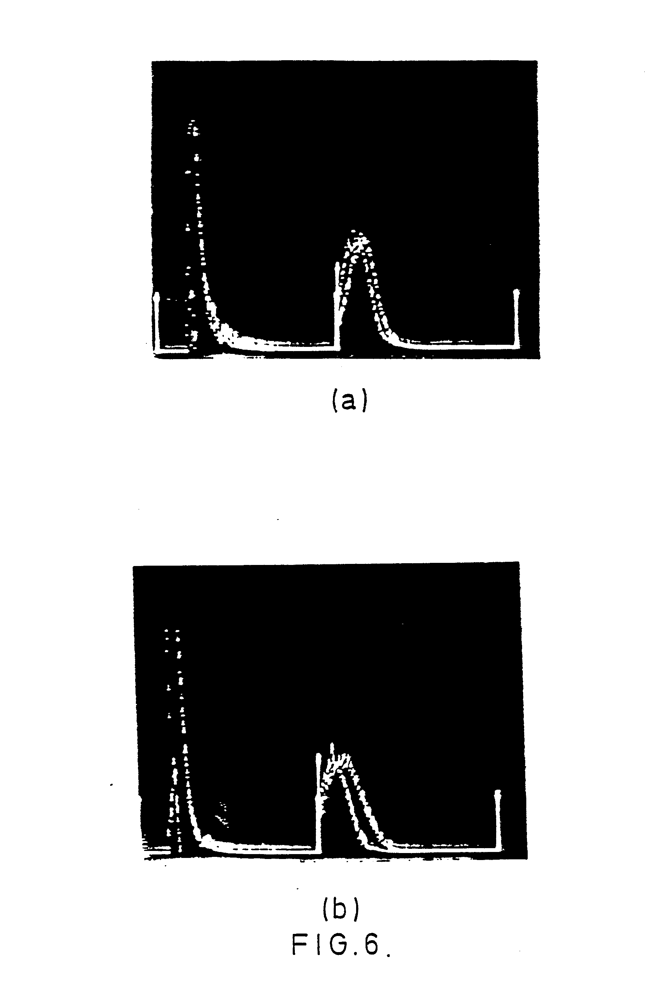

Live-cell staining of CEM cells with antisera from mice immunised with either of the MAbs, or of CEM/CBL1 cells with antisera from MT151-immunised mice, was equivalent to that with normal mouse serum. Sera from the mice immunised with Leu3a, however, did stain CEM/CBL1 cells at 1:10 dilution. Results are shown in Figure 6. In Figure 6(a). CEM was 1%, CEM/CBL1 7%, and in Figure 6(b). CEM 1%. CEM/RF 23%. To confirm that the sera from Leu3a mice were reacting with a virus-specific antigen, they were used to immunoprecipitate from $^{35}$S-methionine-labelled CEM cells, either uninfected or infected with HIV isolates CBL1 or HTLV-IIIRF. Bands with a molecular weight of 120kD were specifically precipitated from HIV-infected CEM. No band of this molecular weight was seen in the control precipitate from uninfected CEM.

The antisera were also evaluated in the syncytium inhibition assay. Neither sera from MT151 (a MAb recognising an epitope of CD4 distinct from that recognised by Leu3a or OKT4a) immunised mice, nor sera from normal mice were able to inhibit the syncytium induction. The data in Table 1, however, shows that sera from the Leu3a immunised mice were able to inhibit all virus isolates tested. H9-ARV-2 was neutralised more effectively than H9/IIIB, and H9 RUT and RF required higher concentrations of antisera to inhibit syncytia.

## TABLE 1

### Anti-Id and Anti-HIV Reactivity of Antisera

| MAb | MOUSE NO. | RIA ANTI-ID BINDING ASSAY | | | | | DILUTION OF ANTISERA GIVING >80% SYNCYTIA INHIBITION | | | |
|---|---|---|---|---|---|---|---|---|---|---|
| | | DILUTION OF ANTISERA | | | | | VIRUS ISOLATE | | | |
| | | 1:10 | 1:20 | 1:50 | 1:200 | 1:250 | IIIB | ARV-2 | IIIRF | RUT |
| MT151 | 1 | 2070 | ND | 1403 | ND | 630 | – | – | – | – |
| | 2 | 2933 | ND | 1248 | ND | 621 | – | – | – | – |
| Leu3a | 1 | ND | 1740 | ND | 186 | ND | 1:80 | 1:160 | 1:40 | 1:40 |
| | 2 | ND | 1820 | ND | 142 | ND | 1:40 | 1:80 | 1:20 | 1:40 |
| NORMAL MICE | 1 | 126 | ND | 154 | ND | 80 | – | – | – | – |
| | 2 | ND | 87 | ND | 51 | ND | – | .1/10 | – | – |

ND = not done. – = no inhibition of syncytia.

## EXAMPLE 2

A. Generation of Monoclonal Anti-Idiotypic Antibodies.

Three to five weeks old BALB/C mice were immunized intravenously with 30μg/mouse of purified Leu-3a MAb (Becton-Dickinson, Mountain View, CA), prepared according to (i) or (ii) below. Six injections were given given at weekly intervals. Three days after the last injection, the mice were sacrificed and their spleen cells were fused with the mouse myeloma cell line NS-1 (Hammerling, G.J., et al, (Ed's), Monoclonal Antibodies and T-cell Hybridomas: Perspectives and Technical Advances (1981), Elsevier/North-Holland, New York, pp563 et seq.)

(i) KLH COUPLING

1. Calculate moles of antibody, EDAC and KLH to be used based on amount of antibody to be coupled:

$$Ab = x \text{ moles}$$
$$EDAC^* = 10,000 \ (x) \text{ moles}$$
$$KLH^+ = x/50 \text{ moles}$$

$$\text{mol. wt. IgG} = 150,000$$
$$\text{IgM} = 900,000$$
$$\text{EDAC} = 191$$
$$\text{KLH} = 1,000,000$$

Ab is diluted in minimal volume volume of BBS or PBS
EDAC is diluted in $dH_2O$
KLH is diluted in BBS or PBS

2. Cool antibody to 4°C
3. Add EDAC. Mix for 30 seconds at 4°C
4. Add KLH. Mix for 2 hours at 4°C. Mix overnight at 25°C
5. Dialyse against BBS
6. Alum Precipitate. Protein concentrate = Ab + KLH

* EDAC (1-Ethyl-3-(3-Dimethylaminopropyl)Carbodiimide), Cat. #E-7750 Sigma
+ KLH (Haemocyanin, Keyhole Limpet) Cat. # 374817 Calbiochem.

(ii) ALUM PRECIPITATION

Solutions

10% AlKSO₄ (Aluminum Potassium Sulfate Cat. # A-601 Fisher) in .005M PBS, PH 6.2, filtered (gives 5.7 mg/ml of alumina).

Procedure

1) Add 0.4 mg alumina/50 $\mu$g protein dropwise to protein solution with stirring at RT. Bring up to 10 ml with PBS.
2) Adjust PH to 6.8 tp 7.3. Becomes very cloudy.
3) Incubate for 2 hr at RT, allowing alum to absorb with slow stirring.
4) Spin at 3000 rpm for 10 min.
5) Check O.D. of supernatant for unadsorbed protein.
6) Wash pellet in .85% saline, spin.
7) Resuspend pellet in PBS to 100 $\mu$g/ml.

B. Enzyme-linked Immunosorbent Assays

The HTLV-III ELISA (Electro-Nucleonics, Inc., Silver Spring, MD) and the LAV EIA (Genetics Systems, WA) were done according to the manufacturers' specifications. Horseradish peroxidase-goat anti-mouse IgG antibodies (Vector Laboratories, Burlingame, CA) were substituted for goat anti-human IgG enzyme conjugate. The ELISA using psoralen and UV-inactivated HIV were done as described previously (Chanh, T.C., et al. (1986), Eur. J. Immunol., in press). Culture supernatant from 7 day HIV (HTLV-III3) infected H-9 cells was concentrated 10-fold and inactivated by exposure (three times) to Psoralen (Calbiochem, San Diego, CA) at a final concentration of 10$\mu$g/ml and UV irradiation (360 nm) for 15 min. The inactivated HIV was passed over a column of Sepharose 4B. The void volume was collected and concentrated for use. The ELISA was performed using microtiter plates coated with 50 $\mu$l of antigens whose concentration was estimated by using an extinction coefficient of 14 for 1% solution at 280 nm.

To determine the binding of HF1.7 to Leu-3a, ascites fluid from HF1.7 or a control monoclonal anti-Id (GB-2) that recognized an idiotype associated with an MAb specific for hepatitis B surface antigen, were fractioned with 50% saturated ammonium sulphate. The resulting immunoglobulin-containing precipitate was resuspended in borate-buffered saline and the concentration of antibody was determined using an extinction coefficient of 14 for a 1% solution at 280 nm. Varying concentrations of the ant-Id MAbs were adsorbed to triplicate wells of microtiter plates. After blocking of nonspecific sites with 10% normal goat serum (NGtS) the wells were reacted with biotinylated Leu-3a or with a control MAb specific for simian virus 40 large tumor antigen (SV40 antigen). The antibodies were biotinylated using a concentration of 7 mg/ml (?) and a 1:1000 dilution in 10% NGtS was employed in the assay. Following 1 hr incubation at 37°C, the unbound antibodies were removed by washing and specific binding was detected using avidin-horseradish perox-idase and ABTS with $H_2O_2$.

## C. Inhibition of Binding of HF1.7 MAb to Leu-3a.

Microtiter plates were coated with 500 ng/well of Hf1.7 MAb purified by adsorption to, and elution from, Protein A-Sepharose 4B (Pharmacia, Piscataway, NJ). After blocking of nonspecific sites, 5 $\mu$g of various inhibitors were added to the anti-Id coated wells for 1 hr. After incubation and washing to remove unbound antibodies, biotinylated Leu-3a at a 1:1000 dilution was added and the ELISA was done as described above.

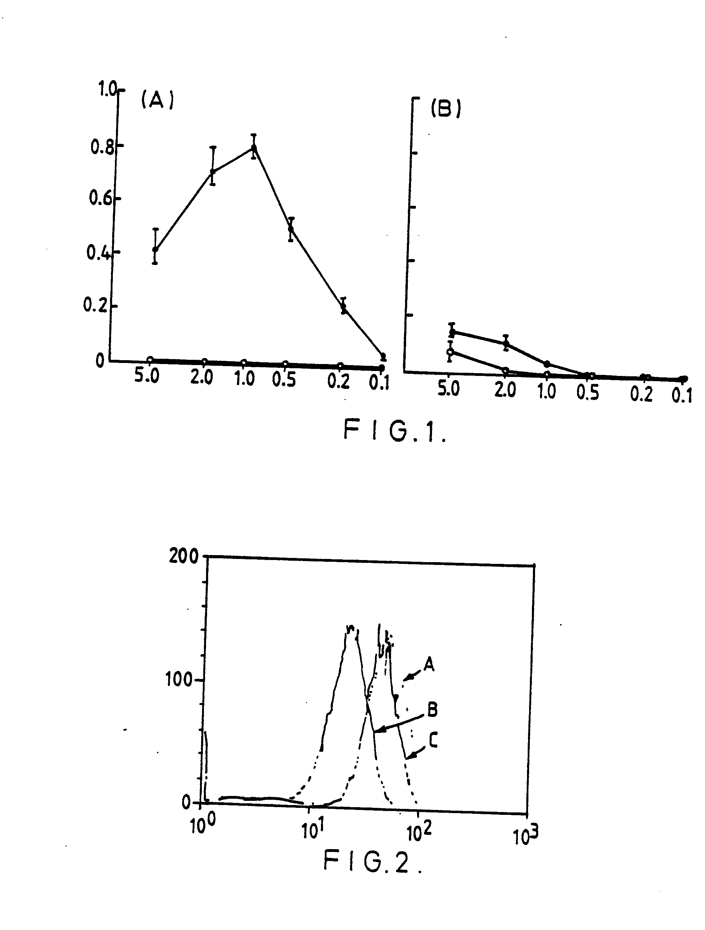

In Figure 1, ascites fluid from TC-1 (●) or a control monoclonal anti-Id (o) that recognized an idiotype associated with an antibody specific for hepatitis B surface antigen, were fractioned with 50% saturated ammonium sulphate. The resulting immunoglobulin-containing precipitate was resuspended in borate buffered saline and the concentration of antibody was determined using an extinction coefficient of 14 for a 1% solution at 280 nm. Varying concentrations of the anti-Id MAbs were adsorbed to wells of microtiter plates. After blocking of nonspecific sites with 10% NGtS, the wells were reacted with biotinylated Leu-3a (A) or with a control MAb sepcific for SV40 antigen (B). The antibodies were biotinylated using a concentration of 7 mg/ml and a 1:1000 dilution in 10% NGtS was employed in the assay. Following 1 hour incubation at 37°C, the unbound antibodies were removed by washing and specific binding was detected using avidin-horseradish peroxidase and ABTS with $H_2O_2$. Each point on the graph represents the mean of triplicate values and the brackets refer to the range. The horizontal axis represents concentration of anti-idiotype in $\mu$g.

## D. Immunofluorescence Staining

Infected and uninfected viable H-9 cells were obtained by centrifugation through Ficoll-Hypaque gradients and washed twice with staining buffer (PBS supplemented with 5% FCS and 0.02% NaN$_3$). One million cells were reacted with HF1.7 anti-Id or a negative antibody control of the same isotype for 30 min at 4°C. The cells were then washed with staining buffer and reacted with FITC-goat anti-mouse IgG (Cappel Laboratories) for an additional 30 min at 4°C. After incubation, the cells were washed, fixed in 0.37% formaldehyde and analyzed using a Becton-Dickinson FACS Analyzer interfaced to a BD Consort 30 (Becton Dickinson, Mountain View, CA). To assess the inhibition of binding of Leu-3a to CD4$^+$ cells by HF1.7, the human T cell line CEM A3.01 was used. FITC - Leu-3a was incubated with PBS or 10 $\mu$g of purified HF1.7 or 10 $\mu$l of control anti-Id MAb for 1 hr at 4°C and added to 5 $\times$ 10$^5$ A3.01 cells. The cells were incubated for 30 min at 4°C, washed twice and analyzed on the FACS analyzer.

## E. Neutralization of HIV Infection In Vitro

One thousand or 100 TCID$_{50}$ of HIV in 100 $\mu$l were incubated with 100.m.l of HF1.7 or GB-2 control anti-Id Or culture medium for 1 hr at 37°C. The concentrations of MAbs were adjusted to yield a final concentration of 0.5 mg/ml. After incubation, the treated HIV were added to 10$^6$ A3.01 cells and incubated at 37°C for 2 hrs in the presence of Polybrene at 10 $\mu$g/ml. The cells were then washed and resuspended in RPMI 1640 10% FCS at a density of 10$^6$/ml. At indicated time intervals, aliquots of culture fluids were removed and reverse transriptase activity was determined. Cell-free HIV was harvested from chronically infected A3.01 cell culture, titrated on uninfected A3.01 cells and the titer was expressed as 50% tissue culture infective dose (TCID$_{50}$).

Hybrids were screened using ELISA'S with HIV antigen-coated plates (Table 2). Among 389 hybrids tested, one was found that reacted in all three assays used. This Mab, designated HF1.7, was cloned twice by limiting dilution. Its isotype was determined to be $IgG_{2b}$.

To assess the specificity of HF1.7 binding, microtiter plates were coated with varying concentrations of HF1.7 and the control MAb, GB-2, and reacted with biotinylated Leu-3a (Fig. 1A) or biotinylated control MAb of the same isotype as Leu-3a but recognizing SV40 T antigen (Fig. 1B). HF1.7 anti-Id MAb specifically bound to the biotinylated Leu-3a and the control anti-Id. Neither HF1.7 nor the control anti-Id MAb bound to biotinylated control MAb specific for SV40 antigen. HF1.7 anti-Id did not react with a panel of irrelevant murine MAbs which included Leu-1, Leu-2a, Leu-5b, Leu-8, Leu-M1 and normal mouse IgG.

At a concentration of 5 μg, the irrelevant MAbs failed to significantly inhibit the binding of Leu-3a to its anti-Id (range of inhibition 0 - 5%), (Table 3). Leu-3a and two other MAbs that recognize the CD4 molecule (OKT-4A and T4) were efficient inhibitors of the Id-anti-Id reaction. These data indicate that HF1.7 recognized an Id determinant on Leu-3a. This Id determinant was shared by other MAbs which recognized CD4, but was not present on MAbs that recognized other lymphocyte phenotypic markers. Leu-3a, OKT-4A and T4 all block in vitro infection by HIV, so ability to inhibit the Id-anti-Id reaction appears to correlate with ability to block HIV infection in vitro.

The binding of HF1.7 MAb to Leu-3a was confirmed in another inhibition experiment using flow cytometry. Human T cell line A3-01, approximately 95% of which express surface CD4, was used in an immunofluorescence staining assay with Leu-3a. Incubation of Leu-3a with the HF1.7 anti-Id resulted in a partial but significant decrease in the fluorescence intensity of the Leu-3a staining (Fig. 2, which shows relative cell no. v. relative fluorescence intensity). Leu-3a staining of the A3.01 cells were not significantly affected by prior incubation with the control anti-Id MAb. These data suggest that the anti-Id binds Leu-3a and partially inhibits Leu-3a binding to surface CD4 present on human T-cells. Therefore, the anti-Id must recognize at least a portion of the antibody combining site on Leu-3a. These characteristics also suggest that HF1.7 recognizes an Id determinant associated with the antibody combining site on Leu-3a.

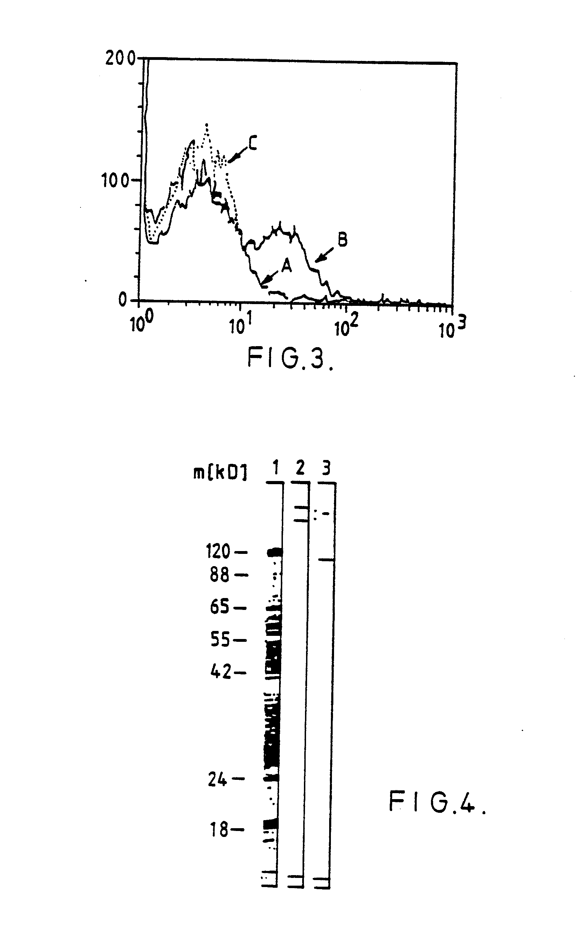

To assess the expression of the antigen recognized by HF1.7 on the surface of HIV-infected cells by the anti-Id, an indirect immunofluorescence assay was performed on uninfected and continuously infected H-9 cells (Fig. 3) which has the same axes as Figure 2). Anti-Id staining of infected H-9 cells resulted in a clear increase in fluorescence intensity whereas uninfected H-9 cells did not stain.

Approximately 25% of HIV-infected H-9 cells were stained by the HF1.7 anti-Id. In order to determine the kinetics of the surface expression of the antigen recognized by the anti-Id on in vitro HIV-infected cells, A3-01 human T cell line was infected with an HIV isolate, designated NY-5, and viable cell membrane indirect immunofluorescence assay with anti-Id MAb was performed on day 1 to day 7 of infection. The antigen recognized by HF1.7 was not detected until day 4 of infection at which point approximately 10% - 15% of the A3.01 cells were stained. Thus, the anti-Id appears to recognize a determinant(s) present on HIV infected T-cells.

To characterize the antigen reactive with HF1.7 anti-Id, nitrocellulose paper strips (Bio-Rad Immunoblot Assay) on which HIV antigens had been electro-blotted were exposed to HF1.7 or to the negative control anti-Id. A pooled human AIDS sera was used as a positive control at a dilution of 1:100. The human antisera recognized the characteristic HIV gag proteins p18 and p24 and the gag precursor p55 in addition to the envelope glycoprotein gp120 and gp41 (Fig. 4). HF1.7 anti-Id reacted with a band corresponding to the gp120 with an approximate $M_r$ between 110-120 kD. No reactivity was found with the negative MAb control. The anti-Id recognized the HIV envelope glycoprotein, gp120, the region where HIV binds the CD4 molecule.

The ability of HF1.7 to inactivate HIV was assessed in an in vitro neutralization assay described in E above. Results are shown in Table 3. Reverse transcriptase activity was inhibited in cultures treated with HF1.7 anti-Id in a viral dose dependent fashion. The most pronounced inhibition of viral relication was observed on day 7 of culture in which 58% and 90% inhibition of Reverse transcriptase activity was observed with 1000 and 100 $TCID_{50}$ of HIV, respectively. By day 9 of culture, the reduction of reverse transcriptase activity in HF1.7 treated cultures declined to 44% and 80% with 1000 and 100 $TCID^{50}$ of HIV, respectively. In contrast, GB-2 treated cultures produced approximately the same reverse transcriptase activity as that detected in medium treated cultures. The increased reverse transcriptase activity in cultures treated with TC1 MAb on day 9 of culture probably resulted from replication of HIV which escaped inactivation.

## Reactivity of TC-1 MAb with HIV Antigens in ELISA

| MAbs | HTLV-III ELISA | LAV ELISA | Psoralen and UV Inactivated HIV |
|---|---|---|---|
| Negative control anti-Id | 0.05 ± 0.01 | 0.06 ± 0.01 | 0.04 ± 0.01 |
| TC-1 anti-Id | 0.75 ± 0.08 | 1.20 ± 0.1 | 0.45 ± 0.03 |

## TABLE 3

## Neutralization of HIV Infection In Vitro by TC-1 MAb

| Viral Dilution | Reverse Transcriptase (cpm) | | | |
|---|---|---|---|---|
| | Day 7 | | Day 9 | |
| | GB-2 | TC-1 | GB-2 | TC-1 |
| $10^{-1}$ | 30,110 | 12,760 (58) | 161,058 | 91,026 (44) |
| $10^{-2}$ | 3,569 | 369 (90) | 53,476 | 10,836 (80) |

EXAMPLE 3

Preparation of OKT4a Anti-Idiotype Antibodies

Mice were immunised, as described previously, with OKT4a prepared by the method of Example 2(A)-(i) and also by the method of Example 2(A)(i) with further coupling to KLH by the use of glutaraldehyde. The resulting sera were tested on HTLV-III ELISA (Electronucleonics) at $OD_{492}$. At dilutions of 1:400, positive results were only observed for mice immunised with OKT4A untreated by guitaraldehyde.

Two MAb cultures were obtained from the successfully immunised mice which gave ELISA readings at $OD_{492}$ of >0.5 for binding both HIV and recombinant gp160.

The 2 MAbs also inhibited binding of OKT4A ($OD_{410}$ = 0.24) but not OKT4B or Leu2a ($OD_{410}$ 0.04 and 0.03 respectively). These tests were performed as previously described.

EXAMPLE 4

Immunisation of Baboons with OKT4a

Two baboons were immunised with alum-precipitated OKT4a 3 times biweekly followed by 2 more times monthly. HTLV-III ELISA (Virgo) at $OD_{492}$ yielded readings of 0.14 and 0.19. For recombinant gp160 at $OD_{410}$ the corresponding readings were 0.10 and 0.14. These readings compare very favourably with the negative control and pre-immune serum ($OD_{492}$ 0.02 and 0.04, $OD_{410}$ 0.02 and 0.03).

The baboon immune sera inhibited anti-CD4 staining of T-cell lines including Leu3a and OKT4A but not OKT4, as expected, and stained HIV-infected cells.

EXAMPLE 5

Binding Studies

Neutralisation by infectivity inhibition or syncytial inhibition showed neutralisation across all isolates around 1/200 dilution. Results are shown in Table 4.

## Table 4

**A.** Specificity of binding of polyclonal anti-idiotypes (anti-anti-Leu-3a)

|  | Anti-Leu-3a | Dako-T4 | OKT-8 |
|---|---|---|---|
| Mouse 1 | 3,000 | <100 | <120 |
| Mouse 2 | 700 | <100 | <120 |
| Mouse 3 | 5,000 | <100 | <120 |
| Control mouse | <140 | <100 | <120 |

The poorly performing mouse (no. 2) improved with boosting.

### Table 5

B.     <u>By rosetting cells</u>
<u>Specific binding to uninfected cells</u>

No binding to all 6 MAb's.  To infected cells

No. of cells

rosetting      H9-RF

| | No. of cells rosetting | H9-RF |
|---|---|---|
| Mouse 4 | >50% | 1/6144 |
| Mouse 5 | >50% | 1/3072 |
| Mouse 6 | >70% | 1/6144 |
| Mouse 7 | >70% | 1/12288 |
| Negative control | | |
| Mouse | – | – |

Therefore, the above anti-Leu3a idiotypes recognise infected cells and neutralise viral infectivity.

### Table 6

HIV-1 neutralization by anti-anti-Leu-3a
(reciprocal titre)

| | RF | H93B | ARV2 | RUT |
|---|---|---|---|---|
| Mouse 1 | 100 | >100 | >100 | 100 |
| Mouse 2 | 10 | 50 | 10 | 10 |
| (boosting) | nt | >250 | nt | nt |
| Mouse 3 | 100 | >100 | >100 | 100 |
| Control mouse | <10 | <10 | <10 | <10 |
| Mouse 4 | 200 | 250 | 200 | nt |
| Mouse 5 | 200 | 250 | 200 | nt |
| Mouse 6 | 200 | 200 | 200 | nt |
| Mouse 7 | 200 | 200 | 200 | nt |

## Example 6 - Viral Cross-Reactivity

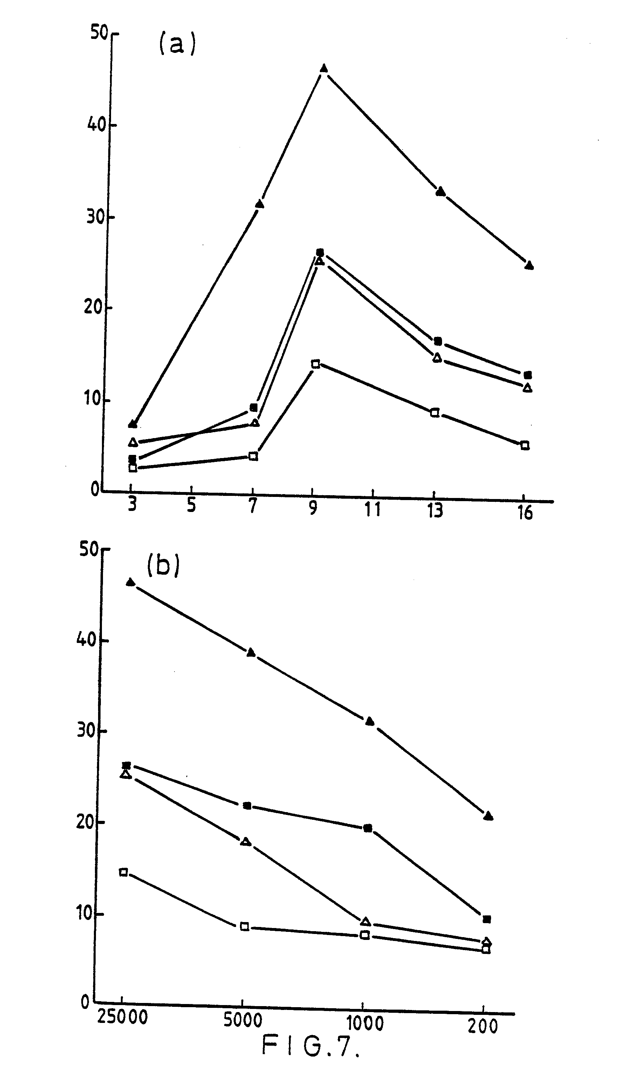

A CD4 mAb (T419THY5D7) which has been shown effectively to inhibit the interaction between HIV-1 and CD4 (12) was chosen to compare the inhibition of infection of peripheral blood lymphocytes with HIV-1 and HIV-2 as measured by subsequent production of reverse transcriptase (RT) activity. Fig. 7a shows that the relative inhibition in RT (CPM $\times$ $10^3$) when cells were treated before infection (HIV (CPM of RT) added at day 0) with the mAb is approximately equivalent in both HIV-1 and HIV-2 infected cultures from days 7 to 16 after infection. Fig. 7b confirms the inhibition in RT activity with both viruses after infection with a range of infectious doses shown as days after infection.

Peripheral blood leucocytes (PBL) were cultured for 3 days in complete medium (RPMI 1640 10% foetal calf serum, 0.2 units/ml anti-interferon serum. and 0.02% polybrene) supplemented with phytohaemagglutinin A (PHA) at a dilution of 1/300. The cells were split into 2 aliquots. One was incubated for 1 hour at 4°C with the CD4 mAb 19THY5D7 at a concentration of 0.05 g/ml, the other without mAb. Supernatants containing HIV-1 or HIV-2 at various conentrations were added to both lots of cells, which were then incubated for 1 hour at 37°C. After washing 3 times, the cells were cultured in complete medium and those preheated with mAb were maintained in 0.1µg of mAb for 6 days of culture. Reverse transcriptase activity was measured in the culture supernatant at various times after infection. ■ = cultures containing HIV-1 only. □ = cultures containing HIV-1 and mAb. ▲ = cultures containing HIV-2 only and △ = cultures containing HIV-2 and mAb.

Figure 7a shows the RT activity in CPM in the cultures over 16 days after infection with supernatants containing $2.5 \times 10^4$ CPM of RT activity.

Figure 7b shows the RT activitiy detected on day 9 after infection with various concentrations of virus.

Syncytium inhibition was carried out with higher concentrations of mAb and was compared after overnight incubation of CD4 bearing cell lines persistently infected with a variety of viruses and virus isolates (Table 7). The majority of mAbs which were tested fall into 2 main epitope clusters, both of which block the HIV-CD4 interaction (12) and comprise group 1. All of these mAbs block all viruses tested with the exception of the D-type Mason-Pfizer monkey virus (MPMV). Group 2 contains 2 mAbs which consistently failed to inhibit syncytium formation with any of the viruses tested. Four other mAbs vary in their ability to block the interaction between HIV-1, HIV-2 and CD4 and only partially inhibited at best. These mAbs were unable to block any of the simian viruses. Vesicular stomatitis virus (VSV) pseudotypes (9) bearing the envelope antigens of HIV-2 and HIV-2 were also tested for their dependence on CD4 receptors for viral binding and penetration.

The results shown in Table 7 were obtained as follows. A panel of 24 CD4 mAbs was used to inhibit syncytium formation between HIV Producing T-cell lines H9, HUT78 or C8166 and the CD4 bearing cell lines C8166 or MT4. Ninety microlitres (µl) of a suspension of the CD4 bearing indicator cell line in RPMI 1640 medium (Gibco) supplemented by 10% foetal calf serum at a concentration of $2 \times 10^5$ cells per ml was added to 10µl of CD4 mAb into each well of a microtitre plate. The CD4 mAbs were either purified immunoglobulin at an original concentration of 100 g per ml or a 1/10 dilution of ascitic fluid with an original titre of at least 1/1000 by immunofluorescence. A further 100µl of a suspension of virus producing cells ($5 \times 10^4$ per ml) was added to each well, and the plate incubated overnight at 37°C. Plates were scored as: - = no inhibition of syncytia (as in wells incubated with no mAb or an irrelevant mAb). + = partial inhibition of syncytia (<20% syncytium formation), + + = complete inhibition. Monoclonal antibodies which consistently blocked all viruses tested with the exception of MPMV were: Leu3a, OKT4a, F101-69, F101-5, EDU-2, NUTH-1, T4, 19thy5D7, 94b1, 91d6, OKT4a, OKT4b, OKT4d, VIT4, MT151, MT321, MT310, T4/18T3A9, 13B8.2 (Group 1).

Those which consistently did not block any of the strains tested were: OKT4 and OKT4C (Group 2).

## TABLE 7

Virus Isolates    CD4 mAbs with variable inhibition of syncytium formation between viruses

| HIV-1 | CLB/10T4 | 66.1 | G19-2 | OKT4e | GROUP 1 | GROUP 2 |
|---|---|---|---|---|---|---|
| H9/HTLV-IIIB | + | + | + | + | + + | - |
| H9/ARV-2 | + | + | + | + | + + | - |
| H9/Ma2 | - | - | + | + | + + | - |

| HIV-2 | | | | | | |
|---|---|---|---|---|---|---|
| H9/LAV-2ROD | + | - | - | - | + + | - |
| H9/CBL2BO | - | - | - | - | + + | - |
| H9/EL6669 | + | - | - | - | + + | - |
| HUT 78/ BK28 (HTLV-IV) | + | - | - | - | + + | - |

| SIV | | | | | | |
|---|---|---|---|---|---|---|
| H9/MAC | - | - | - | - | + + | - |
| H9/MAN | - | - | - | - | + + | - |
| C8166/AGM | - | - | - | - | + + | - |

| D-TYPE | | | | | | |
|---|---|---|---|---|---|---|
| H9/MPMV | - | - | - | - | - | - |

The results in Table 8 indicate the same pattern of inhibition of VSV (HIV) plating for mAbs representative of groups 1 and 2 as seen for syncytium inhibition.

Inhibition of VSV (HIV) pseudotype infectivity by CD4 mAbs. VSV (HIV) pseudotypes were prepared by VSV superinfection of HIV-producing H9 sublines and were titrated on adherent CD4 bearing CCRF-CEM cells pretreated with mAb as described previously (9). VSV plaques were counted 48 hours after pseudotype infection. + + signifies >90% plaque reduction, + represents 40-90% plaque reduction and - indicates no significant plaque reduction.

## Table 8

| VSV (HIV) pseudotype | CB4 mAbs | | |
|---|---|---|---|
| | Group 1 (Leu3a) | Group 2 (OKTA4) | Other (66.1) |
| **HIV-1** | | | |
| HTLV-III RF | ++ | − | + |
| **HIV-2** | | | |
| LAV-2 ROD | ++ | − | − |
| CBL-2 BO | ++ | − | + |
| EL6669 | ++ | − | + |

The cell surface expression of the CD4 antigen on CD4 bearing cell lines uninfected of infected with HIV-1, HIV-2 or SIV was measured by the direct binding of $^{125}$I labelled CD4 mAbs. Table 9 shows that the CD4 mAb was downregulated after infection with any of these viruses, and to a similar extent. This was demonstrated with mAbs to 2 distinct epitopes of CD4. In contrast to these results, infection with the D-type virus which does not use CD4 as its receptor did not affect CD4 expression.

Binding of CD4 mAbs to uninfected and HIV or SIV infected T-cell lines was measured as follows. CD4 mAbs labelled with $^{125}$I (17) were incubated for 30 minutes at 4°C with $2 \times 10^4$ cells in the wells of a 96 well $\mu$-titre plate in the presence of 2% BSA carrier protein (concentration of labelled mAbs were previously standardized to give approximately 1000-2000 bound counts per minute with CEM cells). The plates were washed 3 times, and bound label measured on a gamma counter. The results are expressed as the mean counts per minute in triplicate samples. the specificity of binding of the labelled mAbs was determined by preincubation of the uninfected cells with an excess (20 $\mu$g/ml) of the same, unlabelled mAb.

## Table 9

| Cell line/virus | $^{125}$I labelled CD4 mAb | |
|---|---|---|
| | Leu3a | MT321 |
| CEM | 2121 | 1068 |
| CEM + cold CD4 mAb | 111 | 168 |
| CEM/Ma2 | 87 | 117 |
| CEM/LAV-2$_{ROD}$ | 138 | 105 |
| H9 | 444 | 264 |
| H9 + cold CD4 mAb | 45 | 84 |
| H9/Ma2 | 72 | 99 |
| H9/LAV-2$_{ROD}$ | 49 | 80 |
| H9/CBL2BO | 90 | 78 |
| H9/EL6669 | 66 | 69 |
| H9/STLV-III$_{MAC}$ | 54 | 60 |
| H9/SIV$_{SMM}$ | 42 | 84 |
| H9/MPMV | 300 | 324 |

Example 7 - Use of Mimotypes to Elicit Antibodies

A. Synthetic Peptides and HIV Antigens

The synthesis of peptide 735-752 of gp160 sequences from the HTLV-IIIB isolate was performed by solid phase peptide synthesis (Kennedy. R.C., et al. 1986. Science 231:1556). This peptide corresponds to the second major predicted hydrophilic region in gp41 (Pauletti, D., et al. 1985. Anal. Biochem. 151:540). The amino acid sequence of this peptide is described by Kennedy, R.C., et al. 1986. Science 231:1556.

A control peptide containing the nuclear transport signal recognition site of simian virus 40 (SV40) large tumour antigen (T-ag) was synthesized as previously described (Lanford. R.E., et al. 1986. Cell 46:575). A purified recombinant gp160 peptide produced in the baculovirus expression vector system was used (MicroGeneSys. Inc., West Haven, CT).

B. Antibodies and their Purification

The chimpanzees utilized in this study were part of a synthetic peptide vaccine study for HIV (Kennedy, R.C., et al. 1987. Vaccines 87 Cold Spring Karbor Laboratory, Cold Spring Harbour, N.Y., in press). Two chimpanzees (X-99) and (X-183) received four injections of 200 μg of peptide 735 to 752 coupled to KLH prior to infectious HIV challenge. A control chimpanzee (X-189) received similar injections of the SV40 nuclear transport peptide coupled to KLH. Serum from the individual chimpanzees was pooled following the third and fourth immunizations and prior to challenge with HIV. The first immunization was in Freund's complete adjuvant (FCA) and subsequent injections were in Freund's incomplete adjuvant.

Three New Zealand white rabbits received three biweekly injections of 100 μg of peptide 735 to 752 coupled to KLH in FCA. The specificity of the rabbit antisera has been described Kennedy, R.C., et al. 1986. Science 231:1556; Chanh, T.C., et al. 1986. EMBO J. 5:306). The characteristics of the chimpanzee antisera are summarized in Table 10.

Prior to affinity purification on peptide containing immunoadsorbent columns, the chimpanzee and rabbit immunoglobulin was precipitated with 16% sodium sulfate (w/v). Each globulin preparation was resuspended in 20 ml of borate buffered saline (BBS) and allowed to mix overnight at 4°C with Sepharose 4B or Affi-Gel 501 immunoadsorbents containing peptide. After washing with BBS to remove unbound serum proteins, the anti-peptide antibodies were eluted with 5M potassium iodide. The protein containing fractions were pooled with exhaustively dialyzed against BBS. IgG was purified from resulting anti-peptide preparation by Sephadex G-200 gel filtration. The concentration of immunoglobulin was calculated using an extinction coefficient of 13.5 and 15 for 1% preparation of chimpanzee and rabbit antibody, respectively.

## C. Preparation and Purification of Anti-Id Antibodies

The preparation of xenogeneic anti-Id antibodies in New Zealand white rabbits was by the method of Kennedy, R.C., et al. 1983. J. Immunol. 130:385; Kennedy, R.C., 1983. J. virol. Meth. 7:103. Rabbits were immunized biweekly with chimpanzee X-99 IgG anti-HIV peptide and the resulting antisera were repeatedly adsorbed on a Sepharose 4B column containing preimmune chimpanzee X-99 IgG until all detectable reactivity with isotypic and allotypic determinants was removed. A control anti-Id was produced by immunizing rabbits with chimpanzee X-189 IgG anti-SV40 peptide and subsequent adsorption to remove anti-isotypic and anti-allotypic specificities using preimmune chimpanzee IgG immunoadsorbent columns. The methods for generating these IgG containing immunoadsorbent columns by cyanogen bromide activation have been described elsewhere (Kennedy, R.C. et al. 1984. J. EXP. Med. 159:655). Rabbit IgG was purified from the adsorbed anti-Id containing antisera by using a Staphylococcus Protein A-Agarose column (Schick, M.S., et al. 1987. J. Immunol 138:3419).

## D. Detection of Anti-Immunoglobulin Antibodies

The presence of anti-isotype and anti-allotype, along with anti-Id antibodies, was determined by a direct binding assay using IgG obtained from preimmune and anti-peptide containing chimpanzee serum in a biotin-avidin amplified enzyme linked immunosorbent assay (ELISA) (Schick, M.S., et al. 1987. J. Immunol 138:3419). Two hundred ng in 50 μl of chimpanzee IgG were adsorbed to wells of microtiter plates. Nonspecific sites were blocked and the plate was washed as described above. Rabbit antisera containing the anti-Id were added to the chimpanzee IgG coated microtiter wells. Specific binding was determined using biotinylated goat anti-rabbit gamma globulin and Streptavidin conjugated horseradish peroxidase. The substrates were 2,2'-azino-di-(3-ethyl-benzthiaxoline sulfonic acid) and $H_2O_2$ and the colorimetric reaction was stopped by the addition of sodium dodecyl sulfate. The optical density of each well was read on an automatic ELISA plate reader at 410 nm as previously described (Kennedy, R.C., et al. 1986. Science 231:155617; Schick, M.S., et al. 1987. J. Immunol 138:3419).

## E. KLH Coupling and Alum Precipitation

The methods for coupling anti-Id to KLH, along with alum precipitation were as described in Example 2.

## F. In Vivo Administration of Anti-Id and Peptide 735 to 752 Conjugate KLH

Adult female BALB/c By J mice (Jackson Laboratory, Bar Harbor, ME) were given biweekly injections of 20 µg of IgG anti-Id as an alum precipitate or conjugated to KLH and alum precipitated. Mice were bled 14 and 28 days after the last injection of rabbit anti-Id. Serum obtained from the mice prior to immunization served as a negative control. Mice receiving peptide 735 to 752 conjugated to KLH alone were given three i.p. injections of 20 µg of peptide-KLH as an alum precipitate 14 days apart. These antisera were examined 14 days after the final injection.

## G. Determination of Anti-Peptide and Anti-HIV Activity

The anti-peptide 735 to 752 response in mouse antisera was determined by a biotin-avidin amplified ELISA utilizing a biotin labelled goat anti-mouse IgG (b-gamg; Vector Laboratories, Inc., Burlingame, CA) (Kennedy, R.C., et al. 1986. Science 231:1556). Serum levels of anti-peptide were quantitated as an endpoint titer and represent the last dilution of anti-peptide containing sera that yield a differential $CD_{410}$ of 3 when compared to the $OD_{410}$ obtained with a 1:10 dilution of the preimmune serum.

The anti-HIV activity was similarly determined using a baculovirus gp160 recombinant containing the amino acid sequence associated with HTLV-IIIB isolate. Ten nanograms of recombinant gp160 (MicroGeneSys) was coated onto the individual wells of the ELISA plates in BBS overnight at 4°C. The assay was performed by methods previously described (Kennedy, R.C., et al. 1987. J. Biol. Chem. 262:5769). Anti-HIV titers are similarly expressed as endpoint dilution titers.

## H. Anti-Id Antibody Specificity

To determine the specificity of the rabbit and anti-Id, the following binding assay was employed. Various concentrations of the IgG rabbit anti-Id preparation in 50 µl of BBS were used to coat triplicate wells of a microtiter plate and allowed to adsorb for 1 hour at 37°C. Nonspecific binding sites were blocked with 10% normal goat serum (NGS) and then the plate was washed in phosphate buffered saline containing Tween 20 (T-PBS). Biotinylated chimpanzee anti-peptide IgG preparations, at approximately 10 ng/well, were added and allowed to bind for 1 hour at 37°C. IgG that did not specifically bind the anti-Id were removed by washing the T-PBS. The detection of Id-anti-Id binding was performed as described for the anti-immunoglobulin ELISA.

## I. Inhibition Assays

Inhibition of the Id-anti-Id reaction was used to examine the inhibiting capacity of various chimpanzee and rabbit antibodies or mouse antisera. A constant amount of IgG rabbit antibodies or mouse antisera. A constant amount of IgG rabbit anti-Id (156 ng in 50 ul) was adsorbed to the solid phase for 1 hour at 37°C. This concentration of anti-Id corresponded to the linear part of the Id binding curve. Nonspecific binding sites were blocked by adding 10% NGS for 30 minutes at 37°C and then the plate was washed in T-PBS. Biotinylated-IgG-chimpanzee X-99 Id (b-Id; 10 ng/well) was mixed with different concentrations of various inhibitors and then added to triplicate wells allowed to incubate for 1 hour at 37°C. The remainder of the assay is as described for the anti-immunoglobulin ELISA. Maximum binding (no inhibition) were wells in which b-Id was added without an inhibitor.

In order to ascertain whether the anti-Id could inhibit the Id from binding to either peptide 735-752 or gp160, a competitive inhibition assay was performed. Inhibition of the Id binding either peptide or gp160 was examined in a manner similar to the Id-anti-Id reaction with the following modifications: 200 ng of peptide 735 to 752 conjugated to BSA or 10 ng of recombinant gp160 was adsorbed to the solid phase overnight at 4°C. After blocking with 10% NGS the ELISA plate was washed three times in T-PBS. Biotinylated-Id (10 ng/well) was mixed with an equal volume of different concentrations of the anti-Id and then to triplicate mocrotiter wells. The addition of avidin-horseradish peroxidase and substrate was performed as described in other assays. Inhibition of Id-anti-Id or Id-peptide reactions was calculated with the formula:

Percent Inhibition is equivalent to:

$$100 \times \left[ 1 - \frac{(OD_{410} \text{ with inhibitor} - \text{background})}{(OD_{410} \text{ without inhibitor} - \text{background})} \right]$$

## J. Biotinylation of Antibodies

Five milligrams of purified IgG in 0.5 ml BBS was mixed with 80 $\mu$l of 0.5 M NaHCO$_3$, PH 9.0, 40 $\mu$l of dH$_2$O, and 10 $\mu$l of a 50 mg/ml solution of biotin-N-hydroxysuccinimide ester (Pierce Chemical Co., Rockford, IL) in dry dimethylformamide (J.T. Baker Chemical Co., Phillipsburg, NJ) and incubated for 1 hour at 25°C. The reaction was stopped by the addition of 25 $\mu$l of 1 M NH$_4$Cl and subsequently the mixture was dialyzed overnight against BBS at 4°C. Biotinylated IgG was mixed 1:1 with glycerol and stored at -20°C in the dark.

## K. Preparation of Immunoadsorbents

Peptide 735 to 752 was coupled to cyanogen bromide activated Sepharose 4B. Approximately 3 mg of peptide 735 to 752 was coupled per 1 ml of Sepharose. The amount of peptide that failed to couple was determined in the effluent by absorbance at 275 nm. The coupling efficiency was 92%. In addition, peptide 735 to 752 and the SV40 T-ag transport peptide were also coupled to Affi-Gel 501 (Bio-Rad Laboratories, Richmond, CA). Affi-Gel 501 is an organomercurial derivative which readily forms a mercaptide bond with free SH groups. Since both peptides contained terminal cysteine residues, this matrix was utilised for preparation of peptide affinity columns. The presence of a free reduced sulfhydryl group on the peptide prior to coupling was determined by reaction of free SH with Ellman's reagent (Ellman, G.L. 1959. Arch. Biochem. Biophys. 82:70). If less than the theoretical amount of free sulfhydryl groups was present, the peptides were reduced with either 5 molar equivalents of sodium borohydride at 4°C (Gunsalus, I.C., et al. 1956. J. Am. Chem. Soc. 78:1763) or dithiothreitol (Cleland, W.W. 1964. Biochemistry 3:480). The Affi-Gel 501 was equilibrated in 10 mM NaHPO$_4$, pH 6, and approximately 2 mg of peptide was added in 10 mM NaHPO$_4$ per 1 ml of gel and allowed to mix overnight at room temperature. The matrix was washed with 50 mM Na acetate, pH 5.0, and the amount of peptide that did not couple was determined using absorbance at 275 nm of the effluent. Free functional sites on the gel were saturated with 0.5 mM cysteine. The gel was washed and stored in 50 mM NaPO$_4$, PH 7.0, prior to use. The coupling efficiency for peptide 735 to 752 and the SV40 transport peptide was 62 and 77 percent, respectively. The cyanogen bromide activated Sepharose 4B was used to prepare the chimpanzee X-99 anti-peptide IgG for immunization of rabbits. Based on binding to peptide 735 to 752, gp160 and the anti-Id, no apparent difference was observed with chimpanzee antibody prepared by the two different affinity chromatography matrices.

## L. Western Blot Analysis

Western blot analysis was performed using the Bio-Rad Immunoblot System (Bio-Rad Laboratories, Richmond, CA). Nitrocellulose strips on which electrophoresed HIV antigens had been blotted were blocked with 20 mM Tris-HCl, 150 mM NaCl buffer 9pH 7.4) containing 1% BSA and 0.2% Tween 20. After blocking of non-specific sites, the strips were reacted with pooled human AIDS sera (1:100) or mouse sera (1:10) overnight at 4°C. The strips were washed with Tris-HCl buffer to remove unbound antibodies. Human and

22

mouse antibody reactivities were detected with alkaline phosphatase conjugated goat anti-human and anti-mouse Ig (Sigma, St. Louis, MO), respectively. The substrate used was provided by Bio-Rad Laboratories.

The characteristic of the chimpanzee anti-peptide containing sera relative to binding HIV envelope antigens are summarized in Table 10.

TABLE 10

Characteristics of the Chimpanzee Anti-Peptide Preparation used to Generate the Anti-Id

1. Reacts with HIV gp41 by Western blot.
2. Reacts with HIV gp160 by radioimmunoprecipitation of $^{35}$S-cystine labeled proteins.
3. Binds peptide 735 to 752 coupled to BSA by ELISA.
4. Binds recombinant gp160 by ELISA.
5. Partially reduces reverse transcriptase (RT) activity of HIV in vitro from 42 to 72 percent relative to the reduction of RT by reimmune serum at various input concentrations of virus.

Both chimpanzee antisera reacted with gp41 by Western blot and gp160 by radioimmunoprecipitation. The antisera bound peptide 735 to 752 and the recombinant gp160 in solid phase ELISA. These antisera were not efficient at neutralizing HIV infectivity in vitro and only a partial reduction of HIV reverse transcriptase activity was observed.

Upon challenge with infectious HIV, the chimpanzees developed antibody responses (antibodies to gag proteins) that were indicative of viral replication (Kennedy, R.C., et al. 1987. Vaccines 87 Cold Spring Karbor Laboratory, Cold Spring Harbour, N.Y., in press). Antiserum was selected from chimpanzee X-99 prior to challenge with HIV as the Id preparation to be used for generating an anti-Id.

M. Rabbit Antibody to Chimpanzee X-99 Anti-Peptide IgG Recognizes Id Determinants

In order to demonstrate the anti-Id specificity of the rabbit antibody preparation, binding assays were performed utilizing the Id along with other chimpanzee IgG preparations. Two hundred nanograms of various chimpanzee IgG were bound to the solid phase and various dilutions of the rabbit antibody preparation were added (data not shown). At a dilution of 1:100, the rabbit and anti-Id bound to the chimpanzee anti-peptide IgG idiotype (OD$_{410}$, 0.47), whereas no significant reactivity was observed with IgG from chimpanzee X-99 prior to immunization (OD$_{410}$, 0.02) or with preimmune IgG and anti-SV40 T-ag peptide IgG from chimpanzee X-189 (OD$_{410}$ ; 0.008). In addition, the anti-Id bound chimpanzee X-183 anti-HIV peptide IgG (CD$_{410}$, 0.19) relative to its preimmune IgG preparation (OD$_{410}$, 0.006). These data indicate that the rabbit anti-Id recognized Id determinants associated with a anti-HIV peptide immunogen from two different chimpanzees. This determinant was not present in their respective IgG preparations prior to immunization with the peptide. In contrast, the anti-Id preparation to the anti-SV40 T-ag peptide bound only the Id immunogen (chimpanzee X-189), but not to preimmune IgG or anti-HIV peptide IgG preparation.

N. Rabbit Anti-Id Recognizes the Id Antigen Combining Site

Four types of Ab-2 are produced following immunization with an Ab-1 (Bona, C., et al. 1984. Monoclonal and anti-idiotype antibodies: Probes for receptor structure and function (J.C. Ventar, C.M. Fraser and J. Linstrom, eds.) Alan R. Liss, New York, pp. 141-149). Ab-2α recognizes Id distinct from the antigen combining site. The reaction of this anti-Id is not inhibited from binding to the Ab-1 by the antigen used to induce the Ab-1. An Ab-2β or internal image anti-Id mimics the three-dimensional structure of the antigen recognized by the Ab-1. The Id-anti-Id reaction is inhibited by antigen and these anti-Id bind Ab-1 produced in several different species immunized with the nominal antigen. Ab-2β anti-Id has been demonstrated to contain potential vaccine capacities via the antigen mimicry (Dreesman, G.R. et al, 1985. J. Inf. Dis. 151:761; Kennedy, R.C. 1985. Immunol. 119:1). Ab-2γ also recognize antigen inhibitable Id, but lacks any internal image activity. These anti-Id are usually restricted to certain species or inbred strains within a

23

species and the inhibition by antigen may be due to steric hindrance. The final category of anti-Id is Ab-2ε or epibodies. Such anti-Id recognize an Id and epitope on the Ab-1 and antigen, respectively.

Subsequently, it was examined whether the anti-Id could inhibit the chimpanzee Ab-1 from binding to its peptide 735-752 antigen along with gp160. As shown in Table 11.

## TABLE 11

### Inhibition of Binding of Chimpanzee Anti-HIV Id to Either Peptide 735 to 752 or gp160 by Anti-Id*

| Peptide 735 to 752 | | | gp160 | | |
|---|---|---|---|---|---|
| Inhibitor | Conc. | % Inh. | Inhibitor | Conc. | % Inh. |
| Anti-Id | 10.0 | 47** | Anti-Id | 10.0 | 24 |
| Anti-Id | 1.0 | 25 | Anti-Id | 1.0 | 13 |
| Anti-Id | 0.1 | 16 | Anti-Id | 0.1 | 7 |
| Anti-Id | 0.01 | 9 | Anti-Id | 0.01 | 3 |
| Anti-Id$^C$ | 10.0 | 4 | Anti-Id$^C$ | 10.0 | 1 |
| Anti-Id$^C$ | 1.0 | 2 | Anti-Id$^C$ | 1.0 | 0 |
| Anti-Id$^C$ | 0.1 | 2 | Anti-Id$^C$ | 1.0 | 0 |
| Anti-Id$^C$ | 0.01 | 3 | Anti-Id$^C$ | 0.01 | 1 |

Conc. = Concentration in μg

% Inh. = Percent Inhibition

Anti-Id$^C$ = Control anti-Id

*One hundred nanograms of either peptide 735 to 752 or gp160 was coated to the solid phase and various concentrations of anti-Id were used to inhibit the binding of biotinylated chimpanzee X-99 Id preparation.

**Represents the mean of triplicate determinations.

10 μg of anti-Id could inhibit binding to the peptide by 47%. The control anti-Id failed to inhibit the chimpanzee Ab-1 from this binding. In addition, the anti-Id also inhibited partially the Ab-1 from binding gp160 (24%). Higher concentrations of the anti-Id did not inhibit the Id binding of either peptide or gp160 to a significantly greater degree (data not shown). Together, these data suggest that the anti-Id recognizes a determinant associated in part with the antigen combining site of the chimpanzee Ab-1. The fact that only partial inhibition was obtained suggests that the anti-Id recognizes other determinants associated with the Ab-1 that may not be involved with antigen binding. This is not surprising since the anti-Id is polyclonal in nature and may contain a mixture of the different classes of Ab-2, including some Ab-2$\beta$ or Ab-2$\gamma$, that are responsible for binding to the two chimpanzee anti-HIV peptide ab-1 preparations along with the inhibition of Id to antigen. Non-antigen binding site determinants, along with the fact that the anti-Id bound the homologous Ab-1 preparation better than the heterologous chimpanzee X-183 Ab-1, suggest that the anti-Id may also contain a population of Ab-2$\alpha$. Because the Ab-1 preparation was purified by affinity chromatography and some peptide could have contaminated the Ab-1 immunogen, we examined the ability of the rabbit anti-Id to directly bind peptide 735 to 752 and gp160. No anti-peptide or anti-HIV activity was detectable in the rabbit anti-Id preparation (data not shown). Thus, the inhibition by anti-Id of Ab-1 binding antigen and the detection of a common Id on two chimpanzee anti-peptide preparations does not appear to result from contamination of the Ab-1 preparations by peptide and a subsequent anti-peptide response in the rabbit antibody preparation.

O. Rabbit Anti-Id Recognizes Primarily a Chimpanzee Idiotype

In order to determine if the anti-id possessed internal image activity, we examined the ability of chimpanzee, rabbit and mouse anti-peptide antibodies to inhibit the Id-anti-Id reaction. These experiments were also performed to confirm that isotypic and allotypic determinants were not being recognized in this assay. Only one out of three rabbit anti-peptide preparations and none out of three BALB/c mouse monoclonal antibody preparations inhibited the Id-anti-Id reaction (Table 12).

## TABLE 12

### Inhibition of Binding of Chimpanzee Anti-HIV Peptide Idiotype to Rabbit Anti-Id by Various Inhibitors

| Inhibitors | Specificity | Percent Inhibition |
|---|---|---|
| Chimpanzee X-99 | Preimmune | 1** |
| | Anti-HIV peptide | 85 |
| Chimpanzee X-99 | Preimmune | 5 |
| | Anti-HIV peptide | 28 |
| Chimpanzee X-189 | Preimmune | 0 |
| | Anti-SV40 peptide | 4 |
| Rabbit #1 | Preimmune | 0 |
| | Anti-HIV peptide | 0 |
| Rabbit #2 | Preimmune | 0 |
| | Anti-HIV peptide | 0 |
| Rabbit #3 | Preimmune | 0 |
| | Anti-HIV peptide | 40 |
| Mouse monoclonal[+]#1 | Anti-HIV peptide | 0 |
| Mouse monoclonal #2 | Anti-HIV peptide | 0 |
| Mouse monoclonal #3 | Anti-HIV peptide | 0 |

* Each inhibitor was used at a concentration of 2.5 $\mu$g.
**Represents mean of triplicate determinations.
+ The generation of the mouse moncolonal anti-HIV peptide that stain the surface of HIV infected T-cell lines have been described elsewhere (Kennedy, R.C., et al. 1986. Protides of the Biological Fluids (H. Peeters, ed.) Vol. 34, Pergamon Press, Oxford, P. 107.46).

At a 2.5 $\mu$g concentration, chimpanzee X-99 and chimpanzee X-183 inhibited the Id-anti-Id reaction by 85 and 28%, respectively. Thus, the homologous Id was by far a better inhibitor of the reaction. Confirming the direct binding studies, no significant inhibition was observed with preimmune chimpanzee IgG and chimpanzee anti-SV40 T-ag peptide. Only a single rabbit anti-HIV peptide preparation demonstrated any inhibition of the Id-anti-Id reaction (40%). These data suggest that the anti-Id preparation is not composed primarily of internal image anti-Id due to the failure to recognize a common interspecies idiotype (Kennedy, R.C., et al. 1983. Eur. J. Immunol. 13:232; Sacks, D.L., et al. 1985. J. Immunol. 135:4155). In addition, the homologous Id was the best inhibitor of the Id-anti-Id reaction. This fact, along with inability of some rabbit and mouse monoclonal anti-peptide preparations to inhibit, further suggests that an Ab-2$\alpha$ component might also be present in the anti-Id.

## P. Induction of Anti-HIV Response in Mice Receiving Anti-Id

Previous studies have demonstrated that rabbit anti-Id composed primarily of an Ab-2β or an Ab-2α could induce an anti-hepatitis B surface antigen response in mice without subsequent antigen exposure (Kennedy, R.C. et al. 1984. J. Exp. Med. 159:655; Schick, M.S., et al. 1987. J. Immunol 138:3419). In the following Example, it was examined whether this anti-Id could induce an anti-HIV response specific for peptide 735 to 752.

Four groups of five mice each were given five i.p. injections of rabbit anti-Id as an alum precipitate or coupled to KLH. Two groups received the control anti-Id and the other two received the anti-Id specific for the chimpanzee X-99 Ab-1 preparation. A fifth group of mice were immunized with three injections of peptide 735 to 752 coupled to KLH. Sera from all groups were tested for anti-peptide and anti-HIV activity between 14 and 28 days after the final immunization. In Table 13, the anti-peptide and anti-HIV titers based on binding to recombinant gp160 resulting from anti-Id or peptide immunization are shown. The group that received the rabbit anti-Id to chimpanzee X-99 as an alum precipitate developed both an anti-peptide and anti-HIV response. When compared to mice that received peptide coupled to KLH, the mean reciprocal endpoint anti-peptide titer in the anti-Id treated mice was approximately 3-fold lower (600 compared to 2000). However, the anti-HIV titers between the two groups were comparable (a mean of 400 versus 600). Neither of the two anti-Id immunized control groups showed any anti-peptide or anti-HIV activity at a 1:20 dilution of serum. Thus, the anti-peptide or anti-HIV response did not appear to result from either an anti-rabbit IGG or anti-KLH response in these mice. Immunization with anti-Id coupled to KLH also induced both an anti-peptide and anti-HIV response.

## TABLE 13

### Anti-Id Induces an In Vivo Anti-HIV Response in Mice

| Treatment | No. of injections | Anti-HIV gP41** peptide mean ± SEM | Anti-HIV gP160 mean ± SEM | Inhibition of Id-anti-Id |
|---|---|---|---|---|
| Anti-Id | 5 | 600 ± 200 | 400 ± 200 | 34-67 |
| Anti-Id-KLH | 5 | 300 ± 50 | 150 ± 50 | 30-56 |
| Control anti-Id | 5 | 20 | 20 | 0-6 |
| Control anti-Id-KLH | 5 | 20 | 20 | 0-9 |
| HIV gP41 peptide-KLH | 3 | 2000 ± 500 | 600 ± 40 | 0 |

*Each group of five mice received five injections of 20 μg of either uncoupled of KLH coupled anti-Id as an alum precipitate 14 days apart. Mice were also given three injections of HIV gp41 synthetic peptide conjugated to KLH as an alum precipitate 14 days apart. Mice were bled between 14 and 28 days after the final injection.

**Anti-HIV gp41 peptide or recombinant gp160 antibodies are given as endpoint titers. The data are expressed as the reciprocal dilution of antisera that gave an $OD_{410}$ value greater than three times that obtained with a 1:10 dilution of preimmune sera.

response that does not normally dominate the immune response when antigen is used as the immunogen. The anti-Id response has serological characteristics similar to the Ab-1 used in generating the anti-Id. Sera from control anti-Id (SV40) immunized mice showed no ability to inhibit the chimpanzee X-99 anti-peptide 735 to 752 anti-Id reaction (Table 13). These results indicate that prior injection of rabbit anti-Id leads to the expression of Id positive antibodies that bind gp160 and are specific for peptide 735 to 752.

The activation of clones not normally induced during an immune response, i.e., silent clones, has been shown during the murine immune response to bacterial levan (Bona, C.A., et al. 1981. J. EXP. Med. 153:951; Hiernaux, J., et al. 1981. J. EXP. Med. 153:1004; Sanchez, P., et al. 1985. Mol. Immunol. 22:1231) and HBsAg (Schick, M.S., et al. 1987. J. Immunol 138:3419). Immunization with an anti-Id coupled to KLH induced the production of an anti-levan or anti-HBsAg population which shared an Id with an Ab-1 population that was not usually produced during the immune response to bacterial levan and HBsAg.

In the following Example, the specificity of the anti-Id induced anti-HIV response was determined, and the ability of both peptide 735 to 752 and gp160 to inhibit binding to the peptide was examined (Table 14). At a concentration of 10 μg, the gp41 peptide inhibited binding of the mouse anti-Id anti-HIV to the peptide by 89%. The control SV40 peptide inhibited this reaction by only 6%. At similar inhibitor concentrations, gp160 was a less efficient inhibitor of the peptide-anti-Id anti-HIV reaction when compared to the peptide (52 versus 89%). These data suggest that the anti-Id induced an anti-peptide response that was not completely associated with a gp160 anti-native response. The specificity of the anti-Id induced antibody response appears to contain an anti-peptide and anti-gp160 component.

## TABLE 14

Inhibition of Binding of Mouse Anti-Id Induced Anti-HIV
to HIV gp 41 Synthetic Peptide

| Concentration Inhibitor | Percent (µg) | Inhibition |
|---|---|---|
| HIV gp41 peptide | 10.0 | 89* |
| | 1.0 | 80 |
| | 0.1 | 65 |
| | 0.01 | 40 |
| control SV40 peptide | 10.0 | 6 |
| | 0.1 | 2 |
| | 0.01 | 2 |
| gp160 | 10.0 | 52 |
| | 1.0 | 39 |
| | 0.1 | 21 |
| | 0.01 | 5 |

*Each value represents the mean of triplicate
determinations.

The native component anti-HIV response of the anti-Id induced antibody response was confirmed by Western blot analysis. Anti-Id immunized mouse sera reacted with a band ofmolecular weight 41 kD. No reactivity was seen with the control anti-Id group of mouse sera. A control human serum from an AIDS patient demonstrated reactivity with envelope antigens (160 Kd, 120 Kd and 41 Kd) along with gag proteins (55 Kd, 24 Kd and 18 Kd). These data (not shown) indicate that the anti-HIV response in the mouse antisera is associated with gp41. The reason that the mouse antisera did not recognize gp160 along with gp41 in the Western blot is not known. However, the predominant antibody response in mice and chimpanzees immunized with the gp41 peptide coupled to KLH as assessed by Western blot analysis is to gp41 (Kennedy, R.C., et al. 1987. Vaccines 87 Cold Spring Harbor Laboratory, Cold Spring Harbour, N.Y., in press, unpublished results). The inability of the mouse anti-Id induced antisera to bind gp160 may reflect a relatively specific antibody response directed to a region associated with gp41. Alternatively, the antigen concentration of gp160 in this particular Western blot appears to be optimal. since only a weak anti-gp160 response was detected in the control human AIDS serum. This may be another possible reason as to why the anti-Id induced anti-HIV response recognized only gp41, but not gp160.

## REFERENCES

1. Barre-Sinoussi F. et al Science 220, 868-870 (1983).
2. Popovic, M., Sarngadharan, M.G., Read, E., Gallo, R.C. Science 224, 497-500 (1984).
3. Clavel, F., et al Science 233, 343-346 (1986).
4. Kanki, P.J., et al Science 232, 238-243 (1986).
5. Daniel, M.D., et al Science 228, 1201-1204 (1985).
6. Kanki, P.J., Alroy, J., Essex, M. Science 230, 951-954 (1985).
7. Fultz, P.N., et al Proc. Natl. Acad. Sci. USA 83, 5286-5290 (1986).
8. Guyader, M., et al Nature 326, 662-669 (1987).
9. Dalgleish, A.G., et al Nature 312, 763-767 (1984).
10. Klatzman, D., et al Science 312, 767-768 (1984).
11. Mcdougal, J.S., et al Science 231, 382-385 (1985).
12. Sattentau, Q.J., Dalgleish, A.G., Weiss, R.A., Beverley, P.C.L. Science 234, 382-385 (1986).
13. Weiss, R.A., et al (to accompany this paper).
14. Hirsch, V., et al Proc. Natl. Acad. Sci. USA 83, 9754-9758 (1986).
15. Kornfeld, H., et al Nature 326, 610-613 (1987).
16. Hoxie, J.A., et al Science 234, 1123-1127.
17. Greenwood, F.C., Huneter, W.M., Glover, J.S., Biochem, J. 89, 114-119 (1968).

## Claims

1. A vaccine suitable for use in generating immunity against an AIDS-type virus, comprising an antibody or antibodies, and/or their equivalents, which recognise a surface antigen of said virus, or the cellular receptor therefor, together with a pharmaceutically acceptable, non-toxic carrier.

2. A vaccine according to claim 1, suitable for use in generating passive immunity, the antibodies, or their equivalent, recognising the said antigen.

3. A vaccine according to claim 1, suitable for use in generating active immunity, by generation of an antiidiotype immune response.

4. A vaccine according to claim 3, wherein the antibodies, or their equivalent, recognise the said receptor.

5. A vaccine according to any preceding claim wherein the antibodies, or their equivalent, mimic or mirror the recognition epitope of the CD4 marker.

6. A vaccine according to claim 5 wherein said antibody preparation comprises molecules selected from the group consisting of the monoclonal antibodies Leu3a and OKT4a.

7. A vaccine according to any preceding claim wherein said equivalents are mimotypes.

8. A vaccine according to claim 7 wherein the mimotype is a peptide of gp 120 or gp41, especially the peptide corresponding to the sequence of gp41 between residues 735 to 752.

9. A vaccine according to any preceding claim suitable for generating immunity to all AIDS-type viruses.

10. A vaccine according to any preceding claim further comprising a non-toxic, effective amount of a pharmaceutically acceptable carrier protein and/or adjuvant, especially selected from the group consisting of keyhole limpet haemocyanin and alum preparations.

F I G .1.

F I G .2.

FIG.3.

FIG.4.

FIG.5.

0 287 226

(a)

(b)

FIG.6.

F I G. 7.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | SCIENCE, Research News, vol. 233, 12th September 1986, pages 1149-1153; D.M. BARNES: "Strategies for an AIDS vaccine" <br> * Page 1152, column 3, lines 45-64; page 1153, column 1, lines 1-11 * <br> --- | 1-10 | A 61 K  39/21 <br> A 61 K  39/395 <br> A 61 K  39/42 // <br> G 01 N  33/569 |
| X | FEDERATION PROCEEDINGS, vol. 46, no. 4, 5th March 1987, page 1352, no. 6040; E.M. ZHOU et al.: "Mouse monoclonal anti-anti.cd4, antibodies recognize human immunodeficiency virus antigens" <br> * Abstract * <br> --- | 1-10 | |
| X | FEDERATION PROCEEDINGS, vol. 46, no. 4, 5th March 1987, page 1352, no. 6041; T.C. CHANH et al.: "Anti-idiotypic antibodies against OKT4A bind to human immunodeficiency virus" <br> * Abstract * <br> --- | 1-10 | |
| X,P | BIOLOGICAL ABSTRACTS, vol. 84, 1987, ref. no. 78240, no. 78239, Philadelphia, P.A., US; D.S. LUDWIG et al.: "Anti-receptor antibodies designed to elicit 'internal image'-bearing anti-idiotypes: A possible AIDS vaccine" & MED HYPOTHESES 23(3): 303-308. 1987. <br> * Abstract * <br> --- <br> -/- | 1-10 | **TECHNICAL FIELDS SEARCHED (Int. Cl.4)** <br><br> A 61 K <br> C 12 P |

The present search report has been drawn up for all claims

| Place of search | Date of completion of the search | Examiner |
|---|---|---|
| THE HAGUE | 22-07-1988 | FERNANDEZ Y BRANAS F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P0401)

European Patent
Office

# EUROPEAN SEARCH REPORT

Application Number

EP 88 30 2566

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | PROCEEDINGS OF THE NATIONAL ACADEMY OF SCIENCES USA, vol. 84, June 1987, pages 3891-3895; T.C. CHANH et al.: "Monoclonal anti-idiotypic antibody mimics the CD4 receptor and binds human immunodeficiency virus" * Article * | 1-10 | |
| X,P | THE LANCET, vol. II, no. 8567, 7th November 1987, pages 1047-1050, London, GB; A.G. DALGLEISH et al.: "Neutralisation of hiv isolates by anti-idiotypic antibodies which mimic the T4(CD4) epitope: A potential AIDS vaccine" * Article * | 1-10 | |
| X,P | THE JOURNAL OF IMMUNOLOGY, vol. 139, no. 9, 1st November 1987, pages 2950-2956, The American Association of Immunologists, US; E.-M. ZHOU et al.: "Immune response to human immuniodeficiency virus in vivo administration of anti-idiotype induces an anti-gp160 response specific for a synthetic peptide" * Article * | 1-10 | TECHNICAL FIELDS SEARCHED (Int. Cl.4) |

-/-

The present search report has been drawn up for all claims

| Place of search | Date of completion of the search | Examiner |
|---|---|---|
| THE HAGUE | 22-07-1988 | FERNANDEZ Y BRANAS F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                               
& : member of the same patent family, corresponding document

EPO FORM 1503 03.82 (P0401)

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | THE JOURNAL OF IMMUNOLOGY, vol. 137, no. 9, 1st November 1986, pages 2937-2944, The American Association of Immunologists, US; J.S. McDOUGAL: "Binding of the human retrovirus HTLV-III/LAV/ARV/HIV to the CD4(T4) molecule: conformation dependence, epitope mapping, antibody inhibition, and potential for idiotypic mimicry" * Article * | 1-10 | |
| A,D | SCIENCE, vol. 231, 28th March 1986, pages 1556-1559; R.C. KENNEDY et al.: "Antiserum to a synthetic peptide recognizes the HTLV-III envelope glycoprotein" * Article * | 1-10 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 22-07-1988 | FERNANDEZ Y BRANAS F.J. |

# Marks & Clerk

Incorporating STANLEY, POPPLEWELL, POOLE

European Patent Attorneys
Chartered Patent Agents
Patents Designs and Trade Marks

## 57-60 LINCOLN'S INN FIELDS

## LONDON WC2A 3LS

Telephone 01-405 7636
Telex 25311 EMANDC G
Cables "Marklerk" London WC2
Facsimile 01-404 4910 Groups 2 & 3

The European Patent Office
Postbus 5818
2280 HV Rijswijk
The Hague
Holland

please quote
our reference   GKR/HDL/LW/799P53783

your reference

date   11th July 1988

Dear Sirs

Re:  European Patent Application No. 88302566.0
HIVER Ltd - Application to amend under EPCr. 88

It has been brought to our attention that the application, as filed, was incomplete. Replacement pages 60a and 61 are enclosed in triplicate.

Owing to a computer aberration, 33 lines of text from British Priority Application No. 8725519, filed 30th October 1987, were omitted from the present application. The omitted material should immediately precede the first word on page 61.

We seek to correct the error under the provisions of Rule 88. The wording we seek to insert differs in no way from that of the priority document, and it is submitted that it is immediately obvious that this matter should have been contained in the present application.

We are aware that the technical preparations for publication may have been completed, but we request that the amended pages be included in the specification to be published, if it is at all possible.

Finally, we would point out that we have only/just learned that this omission was made, and have taken steps to remedy the situation without delay.

Yours faithfully

Graham Ruffles
MARKS & CLERK

Enc.